# EUROPEAN PATENT APPLICATION

(11) **EP 4 183 402 A1**
(43) Date of publication of application: **24.05.2023**
(21) Application number: 21842605.4
(22) Date of filing: 15.07.2021
(51) Int. Cl.: A61K 35/28, A61P 25/02

(54) **PLURIPOTENT STEM CELLS EFFECTIVE FOR TREATMENT OF MOTOR NEURON DISEASE (MND)**

(30) Priority: 15.07.2020 US 202063051940 P; 18.09.2020 US 202063080131 P; 15.01.2021 US 202163137780 P
(71) Applicant: National University Corporation Okayama University, Kita-ku, Okayama-shi, Okayama 700-8530 (JP); Tohoku University, Sendai-shi, Miyagi 980-8577 (JP); Life Science Institute, Inc., Tokyo 100-8251 (JP)
(72) Inventor: ABE, Koji, Okayama-shi, Okayama 700-8530 (JP); YAMASHITA, Toru, Okayama-shi, Okayama 700-8530 (JP); DEZAWA, Mari, Sendai-shi, Miyagi 980-8577 (JP); KUSHIDA, Yoshihiro, Sendai-shi, Miyagi 980-8577 (JP); IWASE, Yumiko, Tokyo 101-8517 (JP)
(74) Representative: AWA Sweden AB
(86) International application number: PCT/JP2021/026654
(87) International publication number: WO 2022/014681

(57) **Abstract**

Amyotrophic lateral sclerosis (ALS) is a life-threatening neurodegenerative disease characterized by the progressive loss of motor neurons. Muse cells are endogenous reparative pluripotent-like stem cells distributed to various tissues. Once intravenously injected, such cells, upon sensing sphingosine-1-phosphate produced by damaged cells, settle selectively on damaged sites, and after homing, can exhibit multiphase effects including natural differentiation into tissue-protecting and tissue-reconstructing cells. In the present invention, with G93A-Tg mice serving as an ALS model, human Muse cells injected intravenously successfully homed into the lumbar cord, mainly at the pia mater and the lower white matter, and expressed glial-like forms and GFAP. Meanwhile, no such homing or differentiation was observed in the case of human mesenchymal stem cells (MSCs), and, rather, MSCs were found distributed to the lungs. The Muse group demonstrated significant improvement in the scores of RotaRod, hanging wire, and lower extremity muscle strength, recovered the motor neuron number, and significantly reduced denervation and muscle fiber atrophy in muscles of lower limbs as compared to the vehicle group. Furthermore, the Muse cell effect on motor function of TDP-43 Tg mice serving as an ALS model was evaluated. The results show that, compared to the vehicle group, the Muse cell group had a significantly improved hanging-wire test score. These results indicate that Muse cells perform homing in a damaged-site-dependent manner, and protect the spinal cord in response to the death of motor neurons, and thus Muse cells are useful for treatment of ALS patients.

## Description

### FILED

The present invention relates to a cell preparation for regenerative medicine. More specifically, it relates to an effective cell preparation for treating, preventing, alleviating and/or delaying the onset of motor neuron diseases (MNDs) in subjects, which contains pluripotent stem cells.

### BACKGROUND

Amyotrophic lateral sclerosis (ALS) is a devastating neurodegenerative disease characterized by progressive motor neuron loss. About 10% of ALS patients have a genetically inherited form associated with mutations in Cu/Zn superoxide dismutase (SOD1) [NPLs 1-3] [1-3], TAR DNA binding protein 43 (TDP-43) [NPLs 4 and 5] [4, 5], and a hexanucleotide repeat expansion of the C9orf72 gene [NPLs 6 and 7] [6, 7]. In addition to an oral drug riluzole, a free radical scavenger edaravone was recently approved as a new drug for ALS [NPLs 8 and 9] [8, 9]. However, the therapeutic benefit of those treatments is still greatly limited, which demands a novel therapeutic strategy for ALS.

Multilineage-differentiating stress enduring (Muse) cells are endogenous pluripotent-like stem cells collectable as cells positive for the pluripotent stem cell surface marker, stage specific embryonic antigen (SSEA)-3. They are normally locating in the bone marrow, peripheral blood, and connective tissues of organs and thus are non-tumorigenic [NPLs 10-13] [10-13]. Muse cells are unique because; they recognize damaged tissue and selectively accumulate at the site of damage by intravenous injection because they express the sphingosine-1-phosphate (S1P) receptor 2, which recognizes the S1P produced by damaged/apoptotic cells; after homing to the damaged site, Muse cells replace damaged/apoptotic cells by spontaneous differentiation into the damaged/apoptotic cell-type, and contribute to tissue repair, as shown by animal models of stroke, acute myocardial infarction, epidermolysis bullosa, chronic kidney disease and liver cirrhosis [NPLs 14-18] [14-18]. Besides the tissue repair effects, Muse cells have pleiotropic effects including neovascularization, immunomodulation, trophic-, anti-apoptotic-, and anti-fibrotic-effects [NPLs 18 and 19] [18,19]. Another important uniqueness is that allogeneic-Muse cells escape host immunorejection after intravenous administration and survive in the host tissue as differentiated cells for over 6 months, even without immunosuppressive treatment [NPL 18] [18]. This is partly explained by the expression of human leukocyte antigen (HLA)-G, a histocompatibility antigen that mediates immune tolerance in the placenta [NPL 18] [18]. Based on these properties, intravenously administered allogenic-Muse cells have been applied to clinical trials for acute myocardial infarction, stroke, spinal cord injury, epidermolysis bullosa and neonatal cerebral palsy under approval of the regulatory authority, all without HLA matching or long-term immunosuppressant treatment [NPL 20] [20]. Since Muse cells are able to target damaged tissues, the number of cells required for treatment is at an order of magnitude less than that in mesenchymal stem cells (MSCs) [NPL 21] [21]. For these properties, we examined a possible therapeutic potential of Muse cells for ALS animal model.

### [CITATION LIST]

[NPL 1] Aoki, M. et al. Nat Genet 5, 323-324, doi:10.1038/ng1293-323 (1993).
[NPL 2] Gurney, M. E. et al. Science 264, 1772-1775, doi:10.1126/science.8209258 (1994).
[NPL 3] Rosen, D. R. et al. Nature 362, 59-62, doi:10.1038/362059a0 (1993).
[NPL 4] Arai, T. et al. Biochem Biophys Res Commun 351, 602-611, doi:10.1016/j.bbrc.2006.10.093 (2006).
[NPL 5] Kabashi, E. et al. Nat Genet 40, 572-574, doi:10.1038/ng.132 (2008).
[NPL 6] DeJesus-Hernandez, M. et al. Neuron 72, 245-256, doi:10.1016/j.neuron.2011.09.011 (2011).
[NPL 7] Renton, A. E. et al. Neuron 72, 257-268, doi:10.1016/j.neuron.2011.09.010 (2011).
[NPL 8] Abe, K. et al. Amyotroph Lat Scl Fr 18, 40-48, doi:10.1080/21678421.2017.1361441 (2017).
[NPL 9] Writing, G. & Edaravone, A. L. S. S. G. Lancet Neurol 16, 505-512, doi:10.1016/S1474-4422(17)30115-1 (2017).
[NPL 10] Kuroda, Y et al. Proc Natl Acad Sci USA 107, 8639-8643, doi:10.1073/pnas.0911647107 (2010).
[NPL 11] Kuroda, Y et al. Nat Protoc 8, 1391-1415, doi:10.1038/nprot.2013.076 (2013).
[NPL 12] Tanaka, T. et al. Circ J 82, 561-571, doi:10.1253/circj.CJ-17-0552 (2018).
[NPL 13] Wakao, S. et al. Proc Natl Acad Sci USA 108, 9875-9880, doi:10.1073/pnas.1100816108 (2011).
[NPL 14] Fujita, Y et al. J Invest Dermatol, doi:10.1016/j.jid.2020.05.092 (2020).
[NPL 15] Iseki, M. et al. Cell Transplant 26, 821-840, doi:10.3727/096368916X693662 (2017).
[NPL 16] Uchida, H. et al. Stroke 48, 428-435, doi:10.1161/STROKEAHA.116.014950 (2017).
[NPL 17] Uchida, N. et al. J Am Soc Nephrol 28, 2946-2960, doi:10.1681/ASN.2016070775 (2017).
[NPL 18] Yamada, Y et al. Circ Res 122, 1069-1083, doi:10.1161/CIRCRESAHA.117.311648 (2018).
[NPL 19] Yabuki, H., Wakao, S., Kushida, Y, Dezawa, M. & Okada, Y Cell Transplant 27, 979-993, doi:10.1177/0963689718761657 (2018).
[NPL 20] Dezawa, M. Adv. Exp. Med. Biol. 1103, 305-307, doi:10.1007/978-4-431-56847-6_17 (2018).
[NPL 21] Noda, T., Nishigaki, K. & Minatoguchi, S. Circ J, doi:10.1253/circj.CJ-20-0307 (2020).

### SUMMARY

### [TECHNICAL PROBLEM]

The present invention provides a novel medical use for pluripotent stem cells (for example, Muse cells) in regenerative medicine. More specifically, the present invention provides a cell preparation and/or pharmaceutical composition that include Muse cells and that are effective for treating, preventing, alleviating and/or delaying the onset of motor neuron diseases (MNDs) in subjects, as well as a method of treating the subjects with the above diseases.

### [SOLUTION TO PROBLEM]

The present inventors have found that using a mutant SOD1 (G93A) transgenic mouse model, intravenously (systemically) administered Muse cells accumulated in the damaged spinal cord and reduced denervation and muscle fiber atrophy in muscles of lower limbs, and therefore it allows the treatment and prevention of ALS. Also, the present inventors have found that by injecting or administering Muse cells to an ALS mouse model that overexpresses a ubiquitinated protein (e.g., TDP-43), Muse cells can locally accumulate at the lesion site, differentiate into tissue-constituting cells and repair ALS. Based on these findings, the present invention has been completed.

Thus, the present invention provides the following.
[1] A cell preparation for treating, preventing, alleviating and/or delaying the onset of motor neuron diseases (MNDs) in subjects, comprising pluripotent stem cells positive for SSEA-3 isolated from mesenchymal tissue of a body or cultured mesenchymal cells.
[2] The cell preparation described in [1], comprising a cell fraction wherein the pluripotent stem cells positive for SSEA-3 have been concentrated by external stress treatment.
[3] The cell preparation described [1] or [2], wherein the pluripotent stem cells are CD105-positive.
[4] The cell preparation described in any one of [1] to [3], wherein the pluripotent stem cells are CD117-negative and CD146-negative.
[5] The cell preparation described in any one of [1] to [4], wherein the pluripotent stem cells are CD117-negative, CD146-negative, NG2-negative, CD34-negative, vWF-negative, and CD271-negative.
[6] The cell preparation described in any one of [1] to [5], wherein the pluripotent stem cells are CD34-negative, CD117-negative, CD146-negative, CD271-negative, NG2-negative, vWF-negative, Sox10-negative, Snai1-negative, Slug-negative, Tyrp1-negative, and Dct negative.
[7] The cell preparation described in any one of [1] to [6], wherein the pluripotent stem cells have all of the following properties:
   (i) low or no telomerase activity;
   (ii) having the ability to differentiate into any of three germ layers;
   (iii) exhibiting non-tumorigenic proliferation; and
   (iv) having self-renewal ability.
[8] The cell preparation described in any one of [1] to [7], wherein the MNDs are amyotrophic lateral sclerosis (ALS), primary lateral sclerosis (PLS), spinal muscular atrophy (SMA), progressive muscular atrophy (PMA), or spinal and bulbar muscular atrophy (SBMA).
[9] The cell preparation described in any one of [1] to [8], wherein the pluripotent stem cells have the ability to engraft into the spinal cord.

The present invention can repair motor neuron in a subject suffering from the MNDs, by a tissue-regenerating mechanism in which Muse cells are directly or intravenously administered to the damaged site, and the Muse cells differentiate to normal tissue-constituting cells around the damaged site.

### BRIEF DESCRIPTION OF DRAWINGS

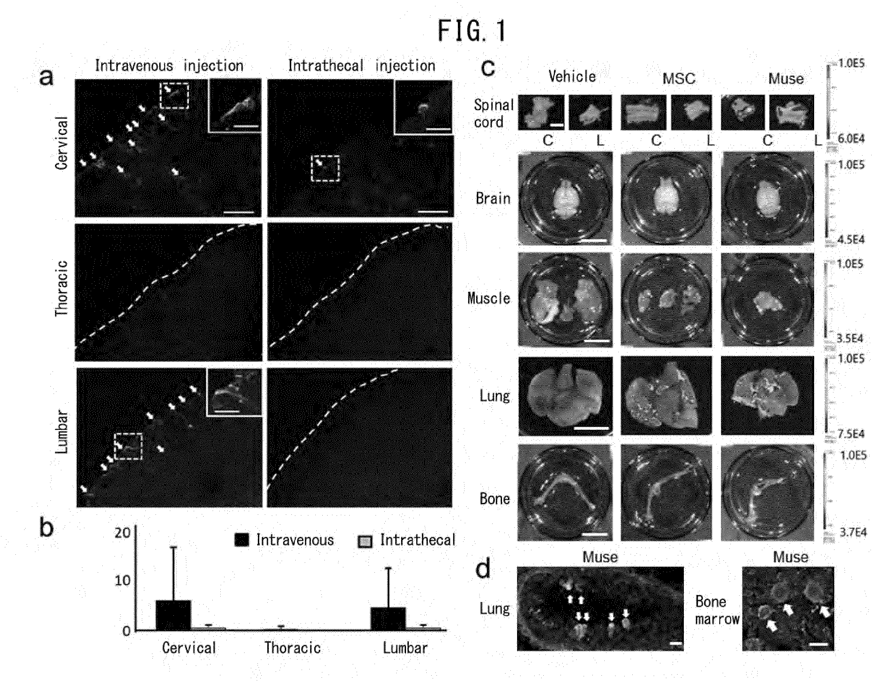

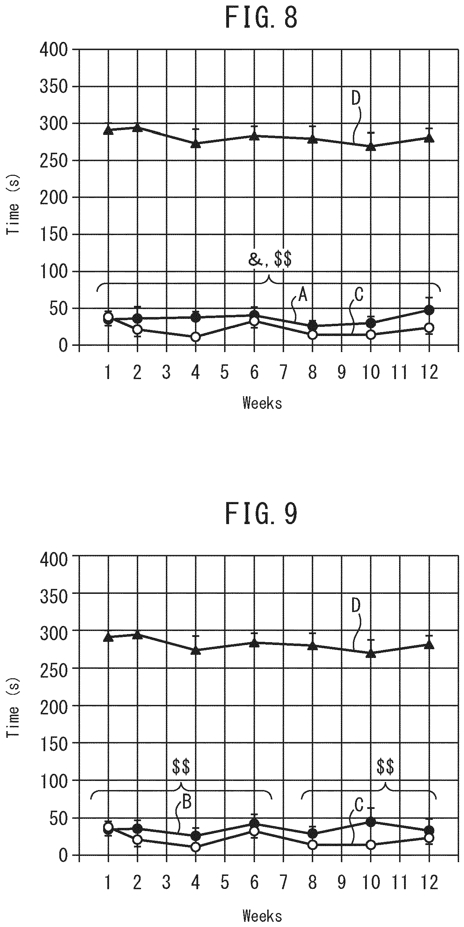

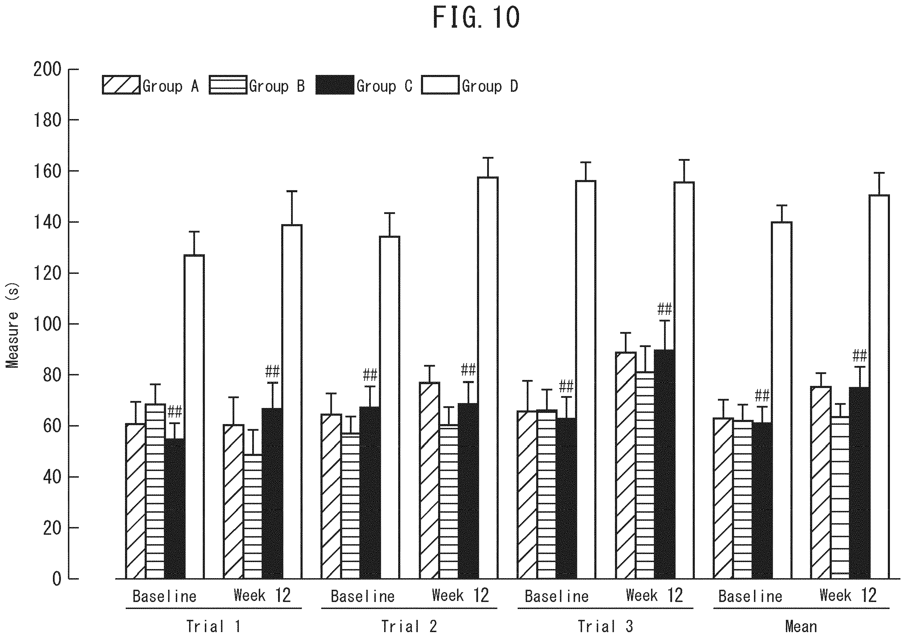

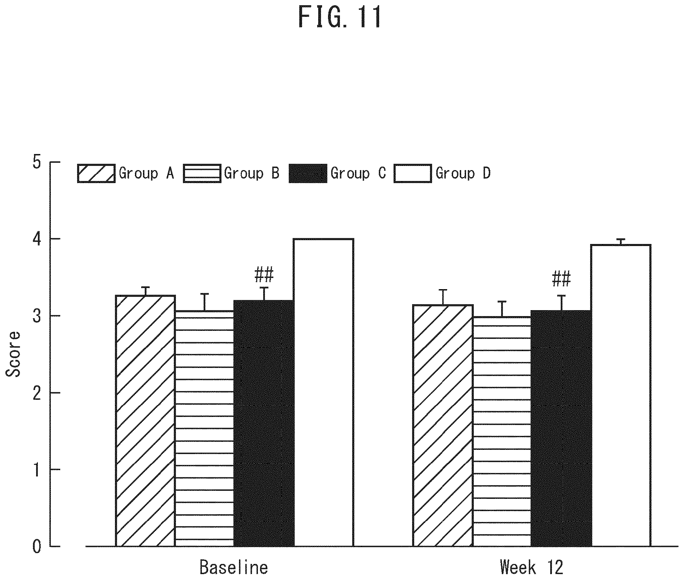

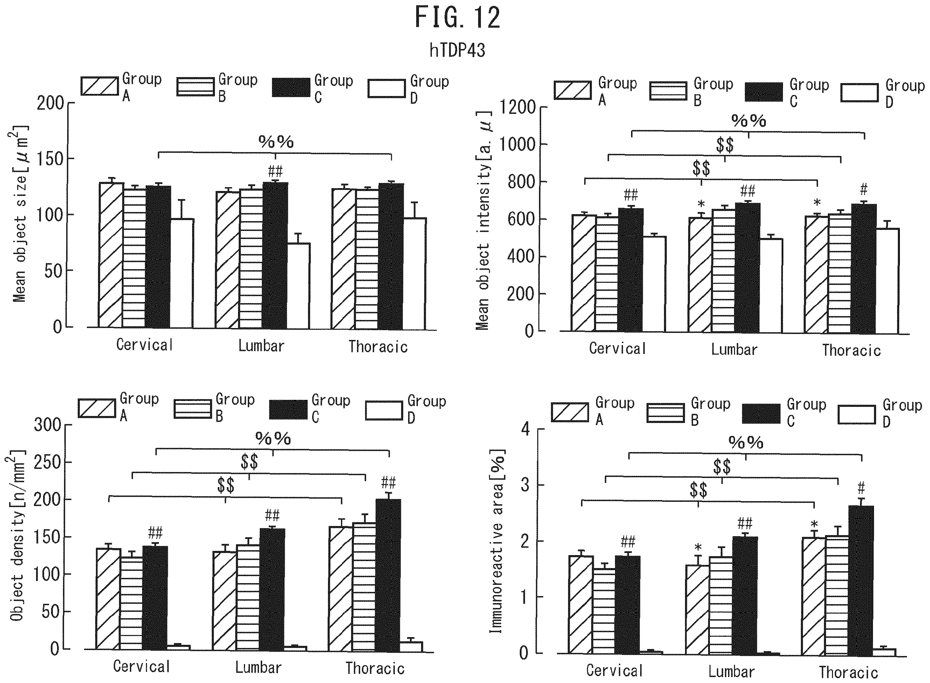

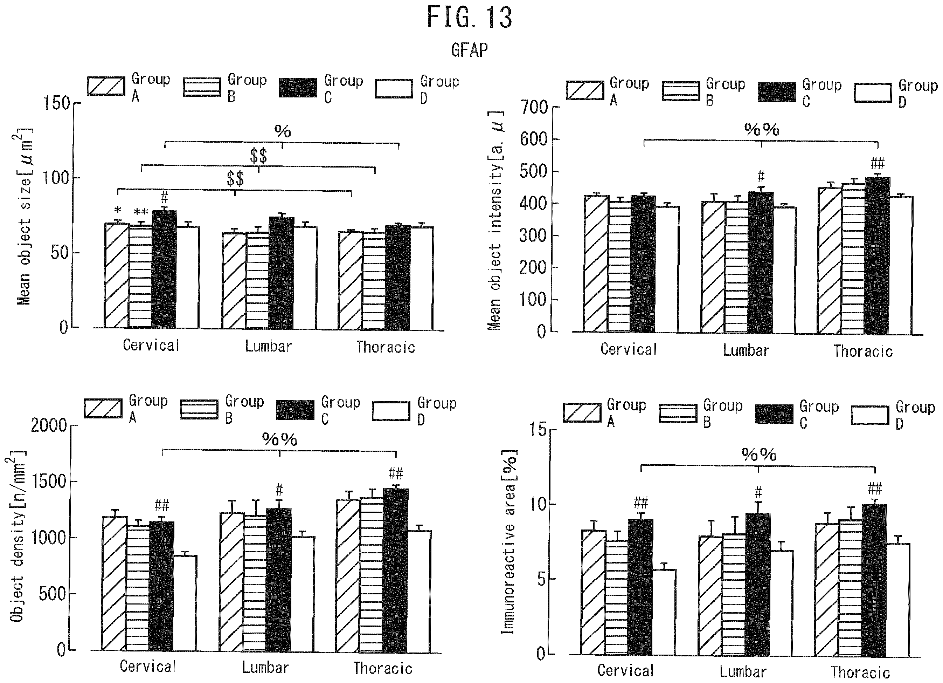

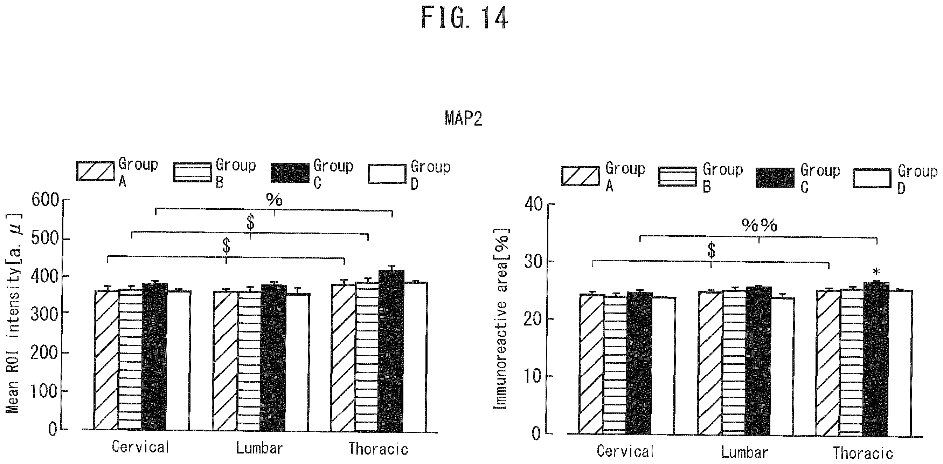

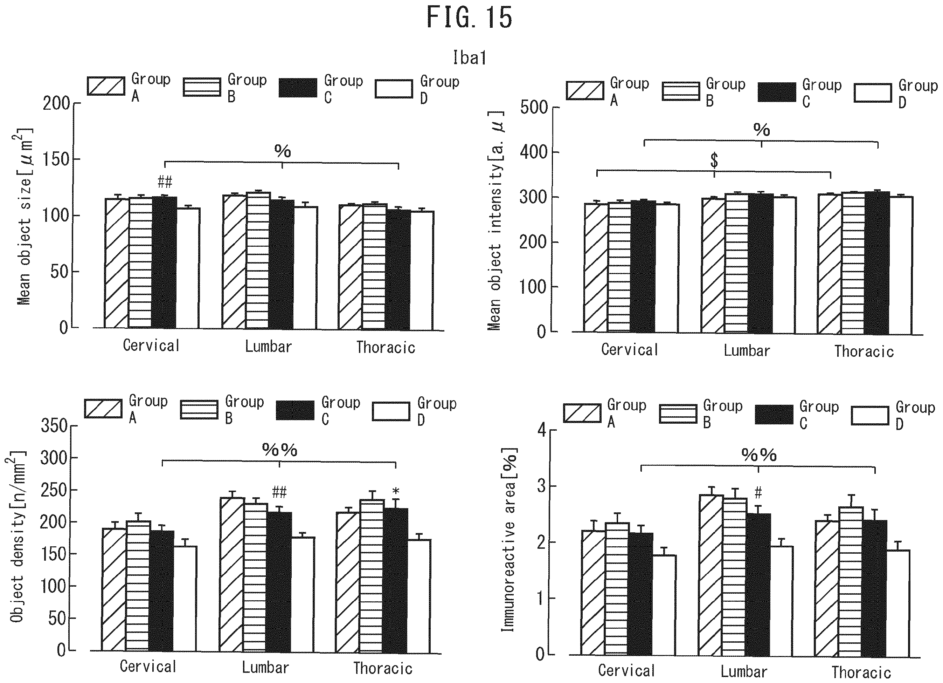

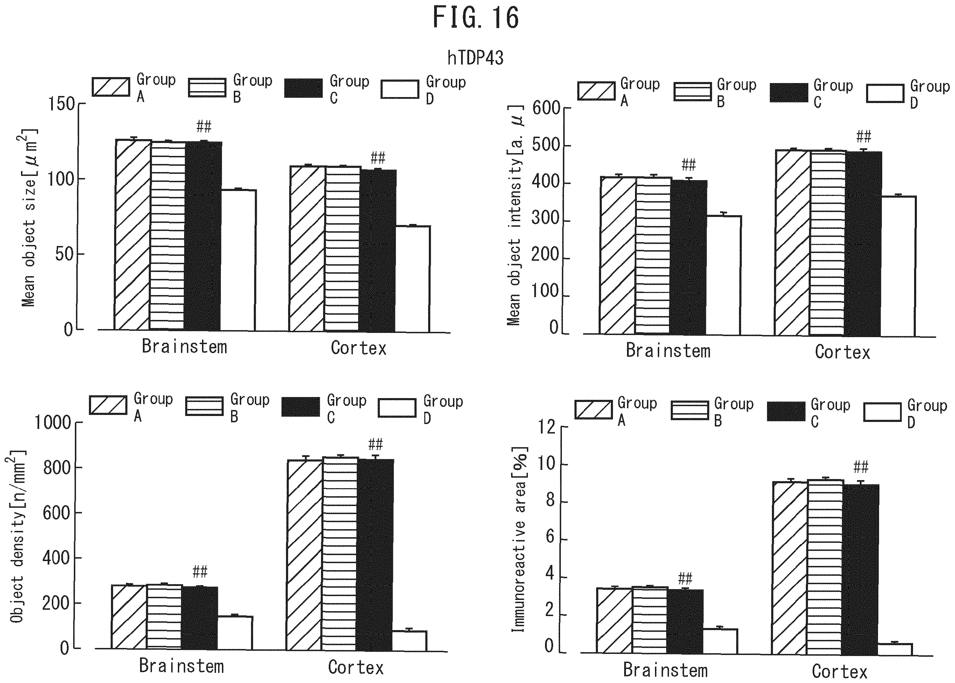

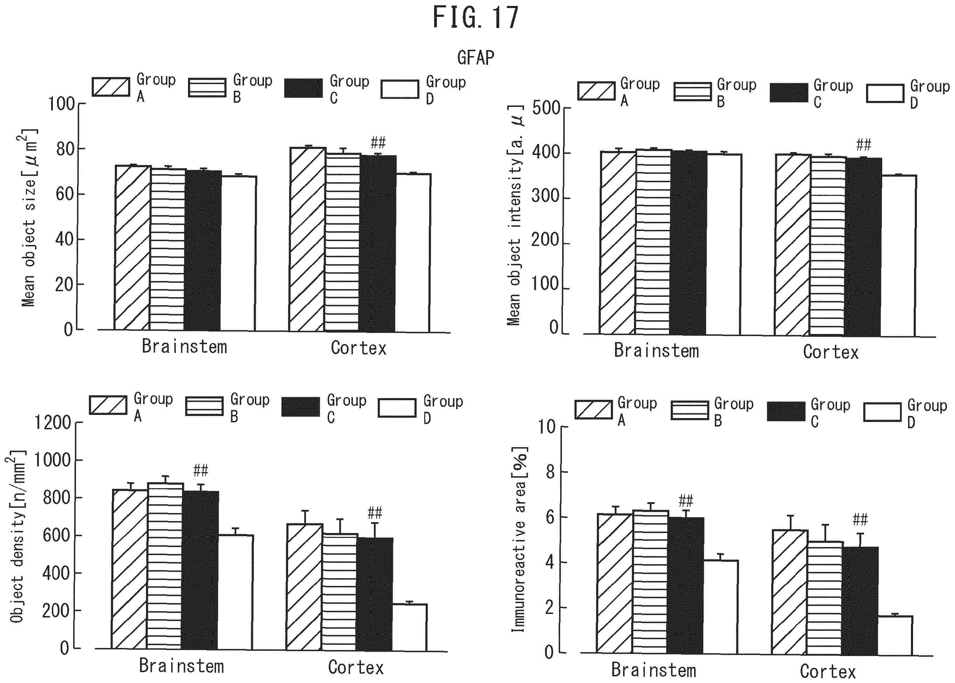

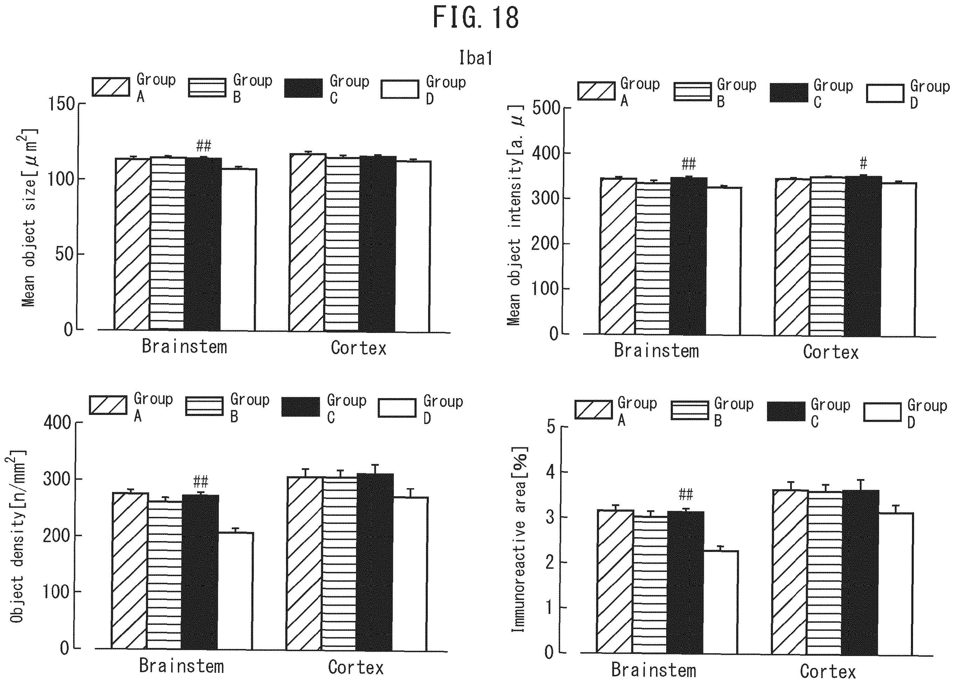

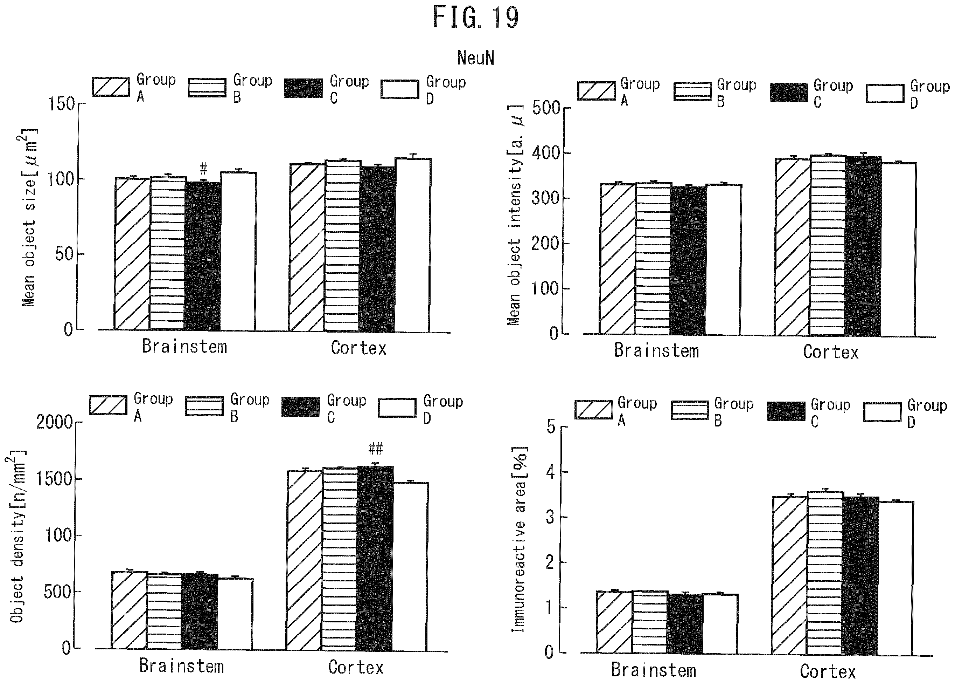

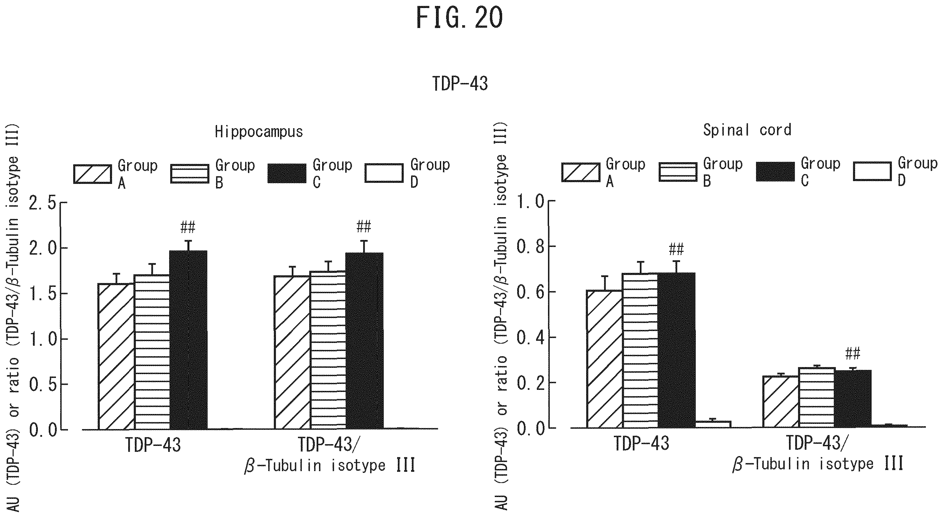

Fig. 1. (a) GFP-labeled Muse cells distribution in the spinal cord at 7 days after intravenous (i.v.) or intrathecal (i.t.) injection. The dotted lined box in panel indicates the high magnification in each panel. Of note, only i.v. injection delivered many GFP-labeled Muse cells to pia-mater and the underneath white matter of both cervical and lumber spinal cords. (b) The number of GFP-labeled Muse cells were higher after i.v. than i.t. injections. (c) Nano-lantern-labeled human MSCs and Muse cells distribution in the spinal cord, brain, muscle, lung, and leg bone at 7 days after the i.v. administration. Of note, only the Muse cells were detected in the spinal cord. (d) Nano-lantern-labeled Muse cells were found in the pulmonary vessel lumen (left panel, arrows), and in the bone marrow (right panel, arrows). Scale bar: (a) 100 µm, (a, bracket) 20 µm, (c, spinal cord) 2 mm, (c, others) 1cm, and (d) 20 µm.

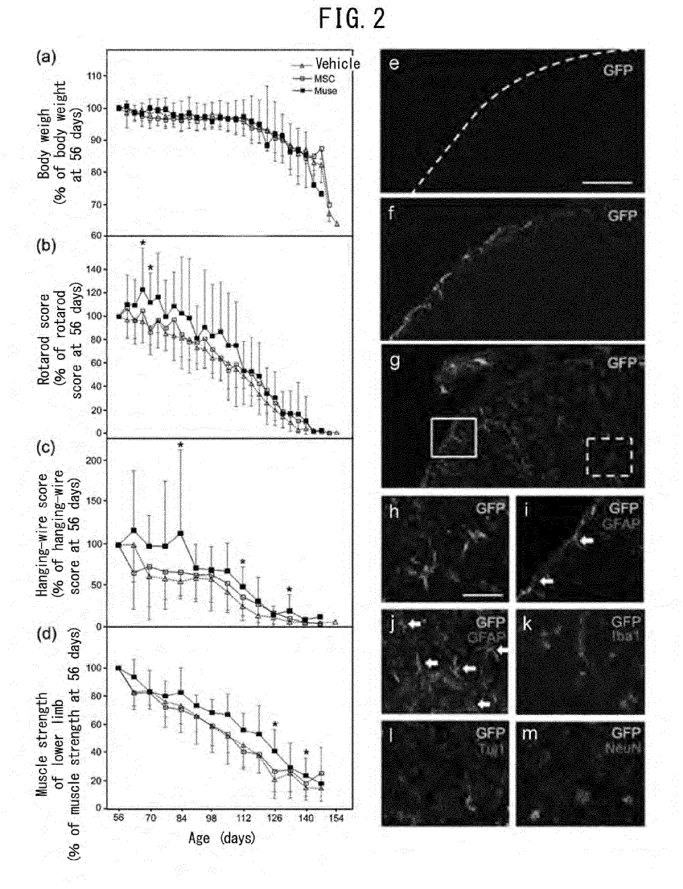

Fig. 2. (a-d) Clinical analysis of G93A mice treated with vehicle (n = 10), MSCs (n = 9), and Muse cells (n = 9) on (a) body weight, (b) RotaRod test, (c) hanging-wire test, and (d) muscle strength of lower limb. Compared with the vehicle, Muse cells i.v. treatment showed significant improvement in RotaRod, hanging-wire, and muscle strength of lower limb (*p<0.05, vs the vehicle). (e) no GFP-positive cell in the lumber spinal cord of G93A Tg mice treated with the vehicle, (f) Several GFP-positive cells were found only in the pia-mater in MSC group, and (g) much more GFP-positive cells detected from pia-mater to ventral horn in the Muse cell group. The boxed area in (g) are magnified in i (solid box) and j (dotted box), respectively. Muse cells treatment showed an evident GFP-positive cells (h) and GFP/GFAP double-positive cells in the pia-mater (i, arrows) and ventral horn (j, arrows) exhibiting morphology typical of astroglia (arrows), but no-double positive for GFP plus microglial marker Iba-1 (k), neuronal marker such as Tuj1 (l), nor NeuN (m). Scale bar: (e) 100 µm and (h) 50 µm.

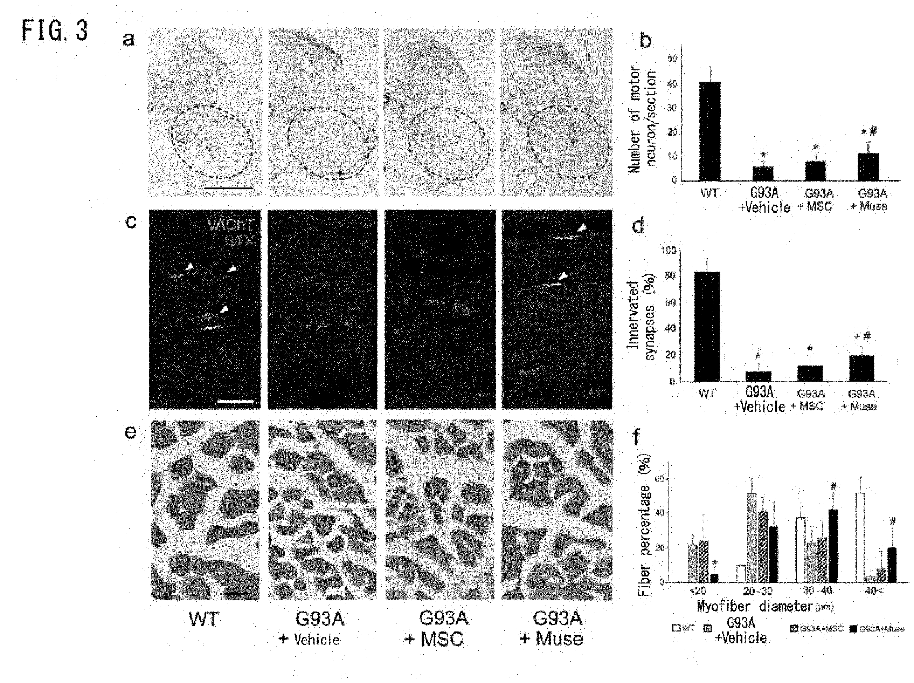

Fig. 3. (a-b) Nissl-stained motor neuron numbers in the lumber spinal cord show the great decrease in G93A Tg mice (*p<0.05, vs wild type = WT), and a significant improvement by Muse cells i.v. treatment (#p<0.05, vs vehicle). (c-d) Neuromuscular junction (NMJ) staining show a denervation in tibialis anterior muscle of G93A Tg mice, and the recovery in Muse cells group (VAChT-positive motor terminals; green, acetylcholine receptors stained with BTX; red, arrowheads, *p<0.05, vs the WT, #p<0.05, vs the vehicle). (e-f) HE-stained neurogenic myofiber atrophy in tibialis anterior muscle of G93A Tg mice, and the significant improvement by Muse cells treatment (*p<0.05, vs the vehicle, #p<0.05, vs MSC). Scale bar: (a) 500 µm, (C) 50 µm and (d) 50 µm.

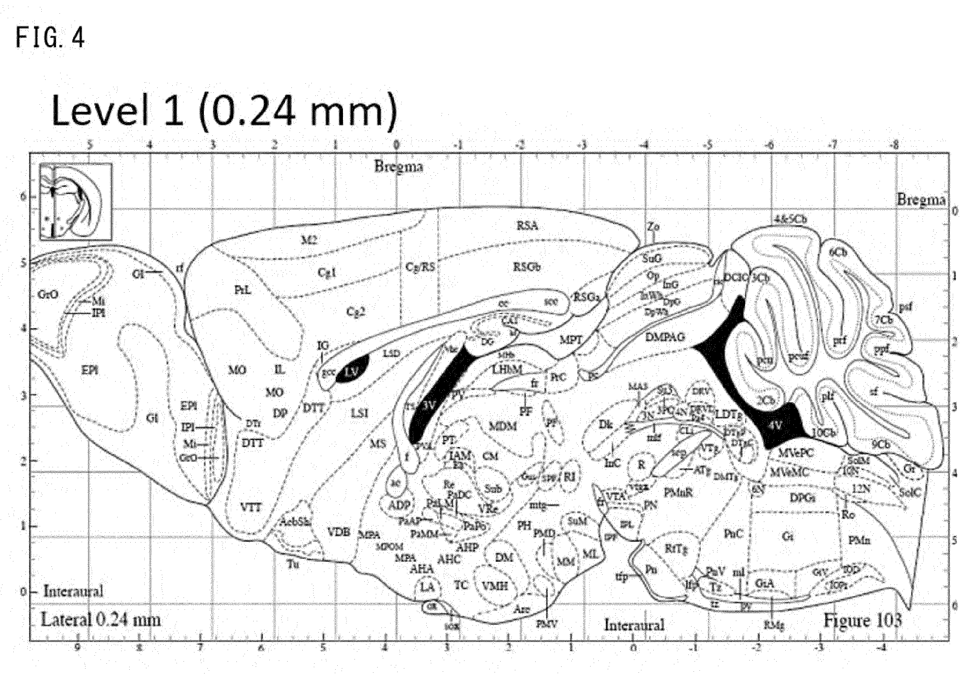

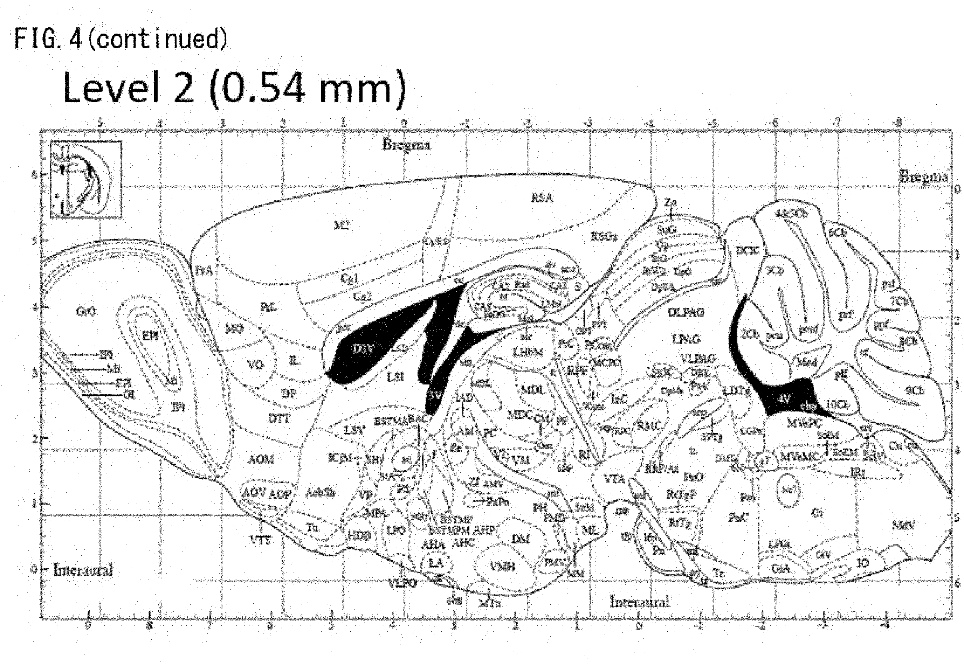

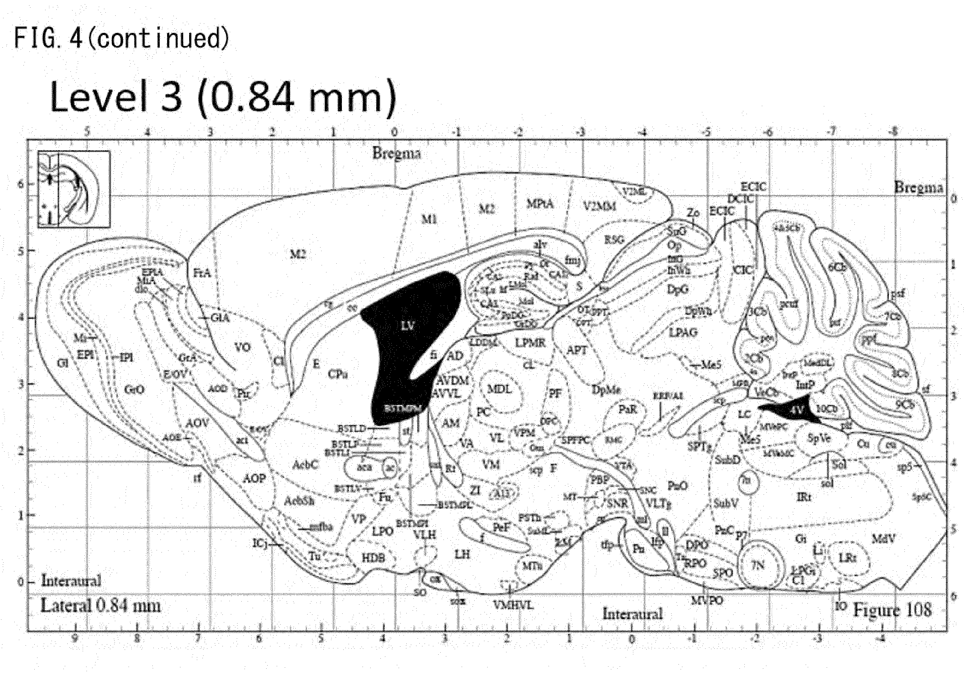

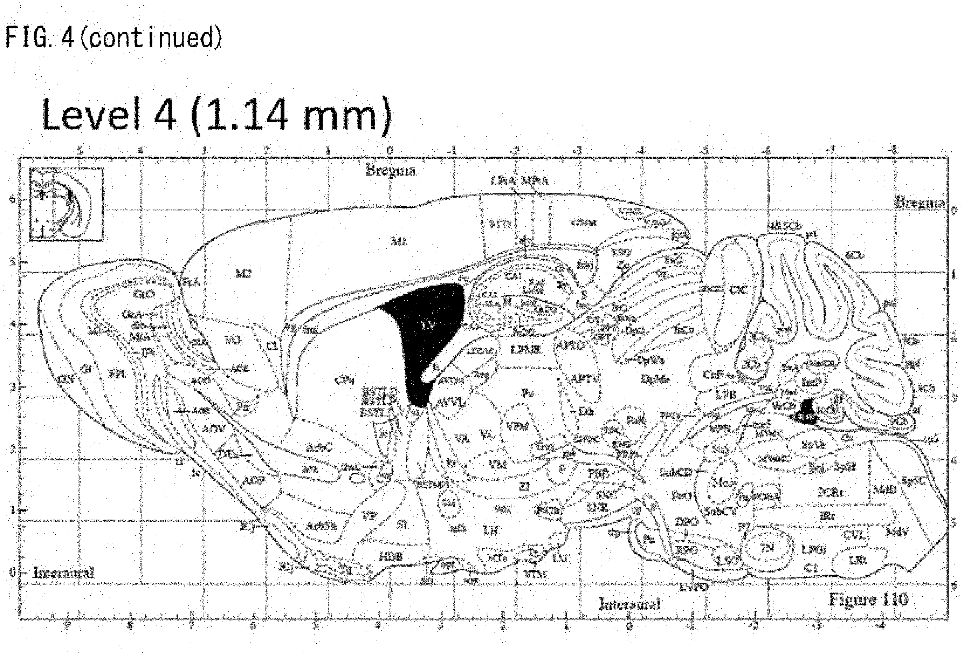

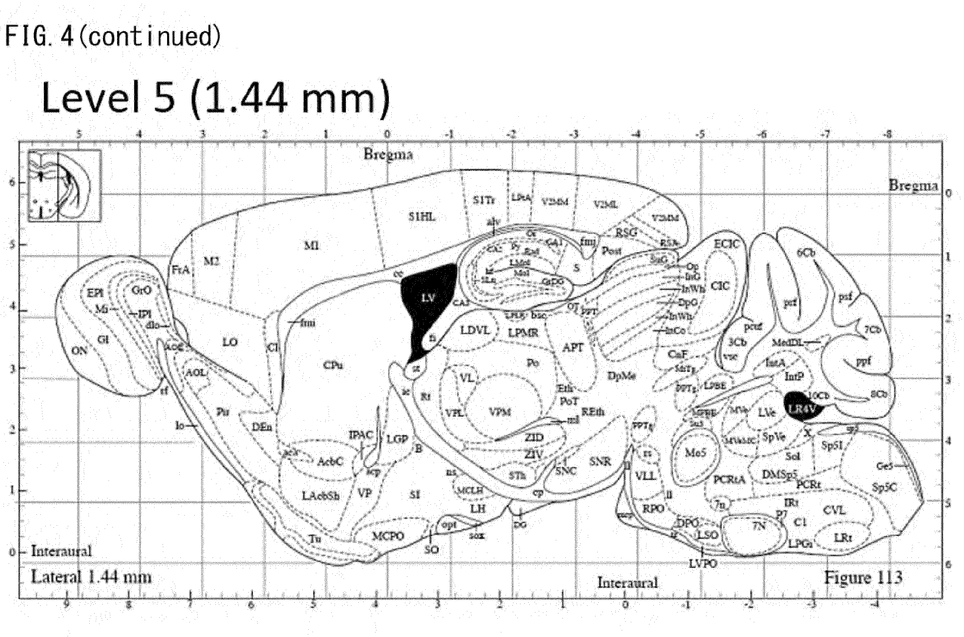

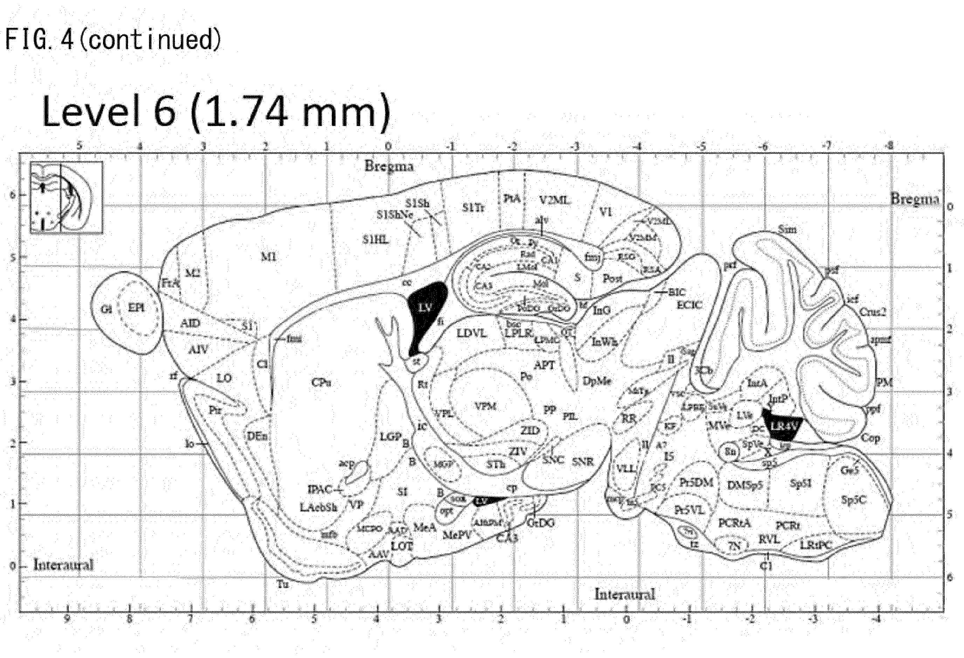

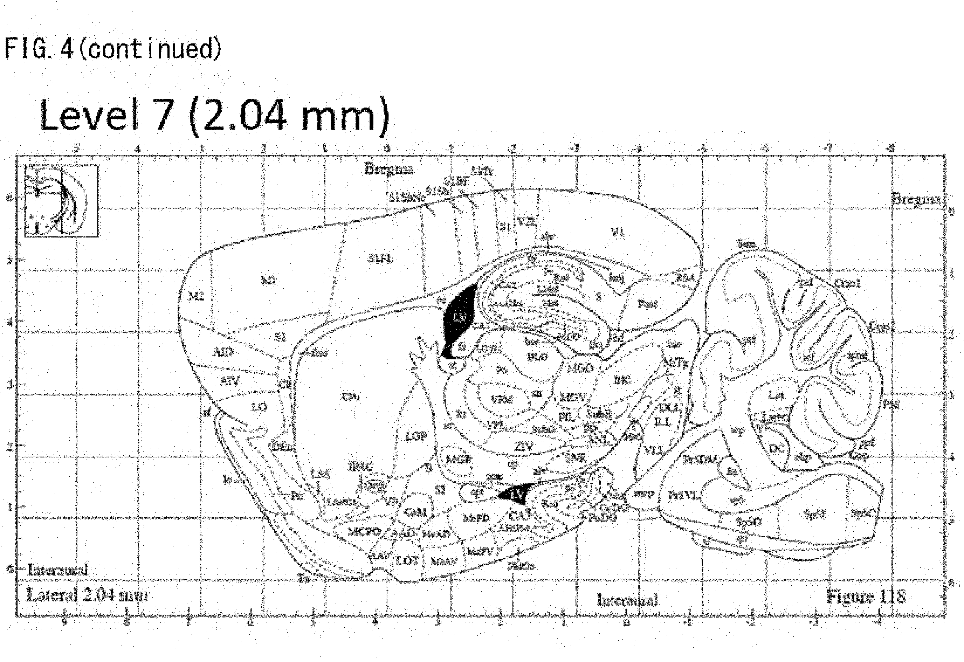

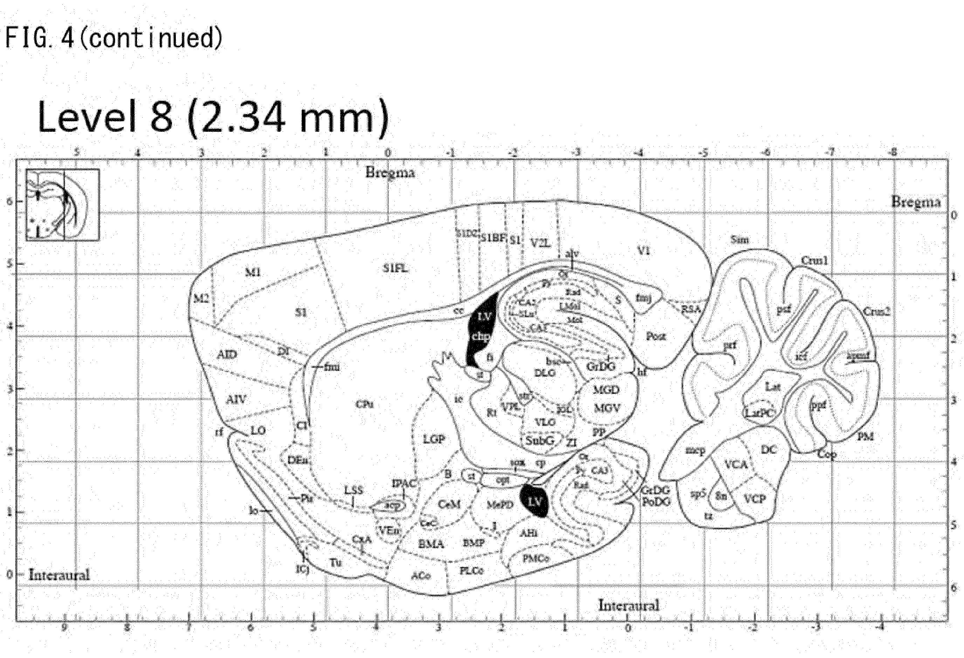

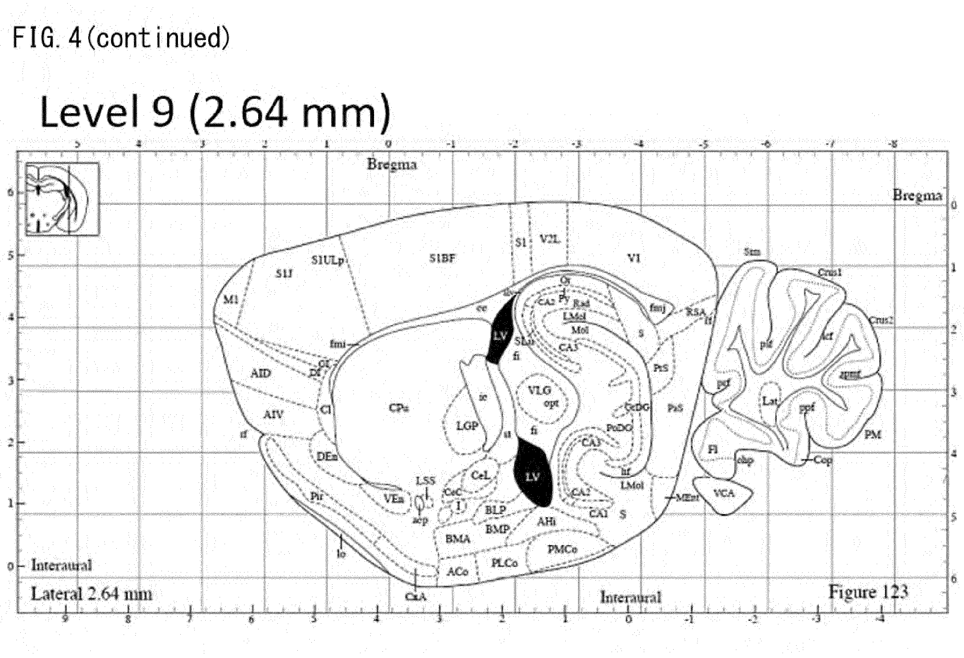

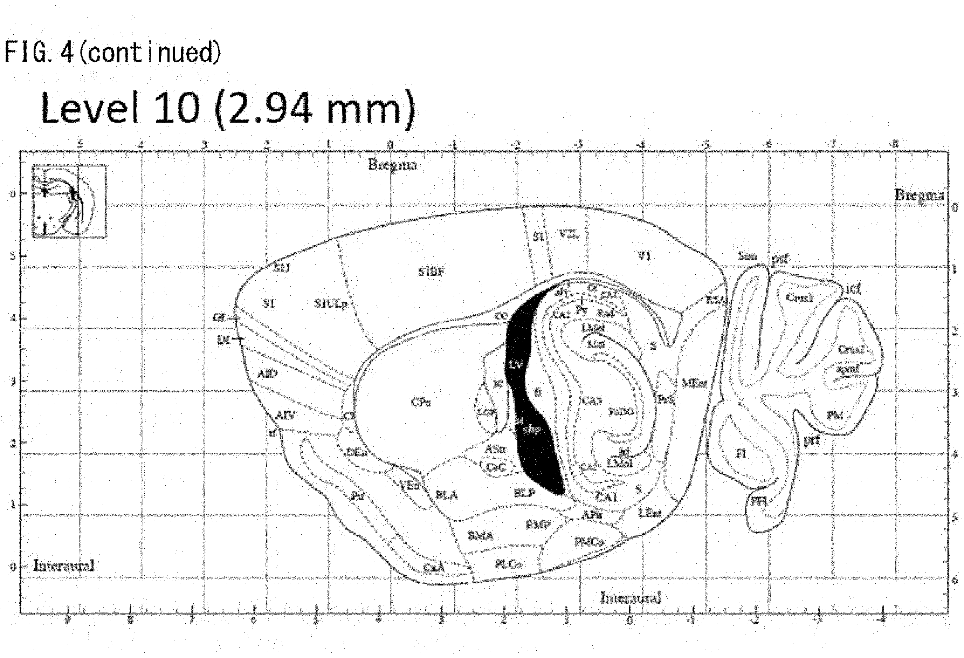

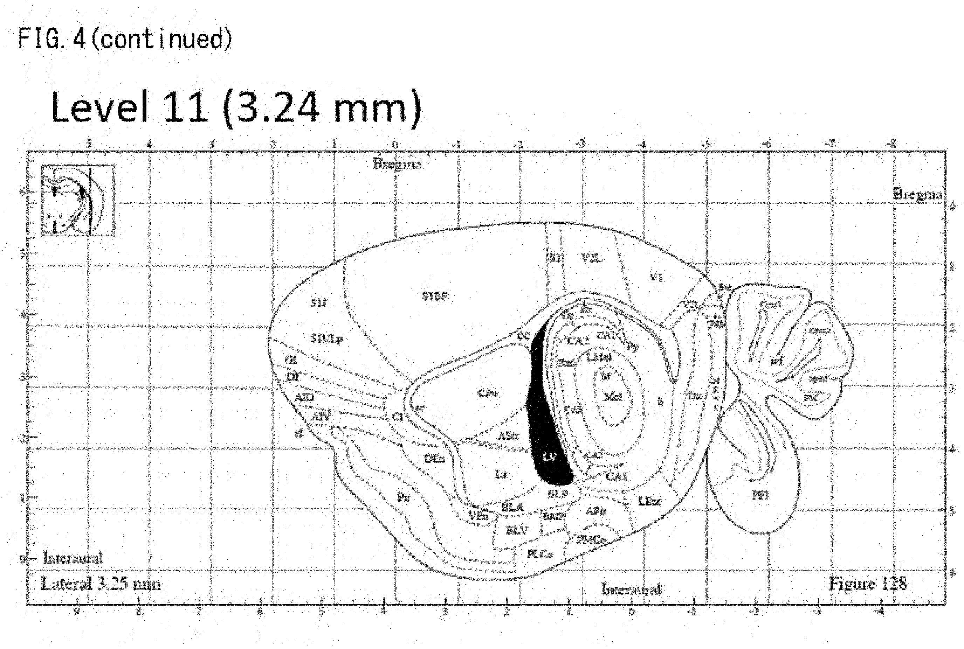

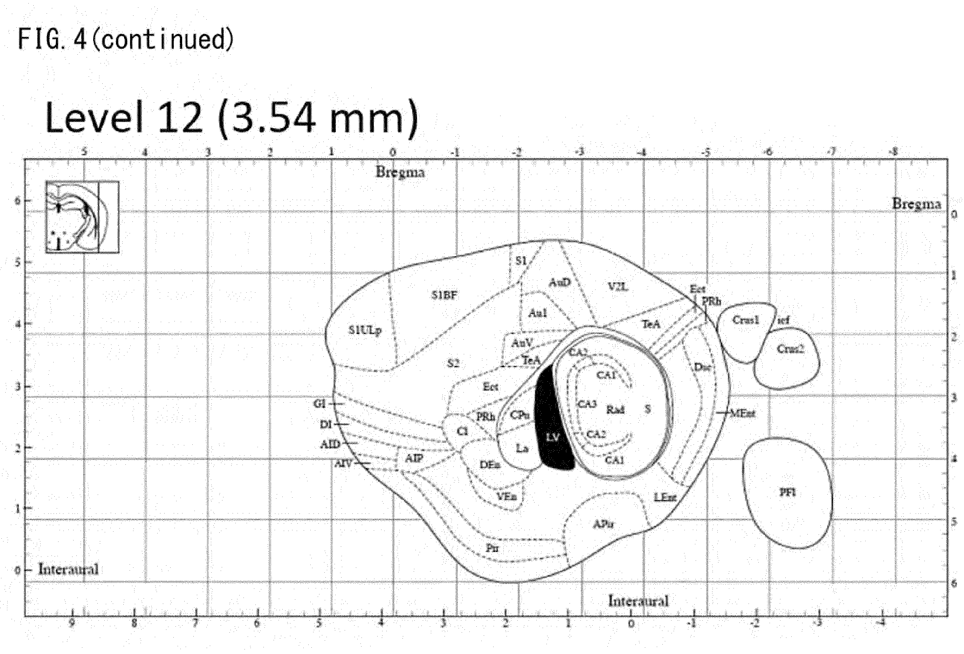

Fig. 4. Sectioning levels. Approximate medio-sagittal levels obtained following the sectioning protocol. Drawings are taken from Paxinos & Franklin "The Mouse Brain Atlas, the 2nd edition", showing the stereotactic coordinates.

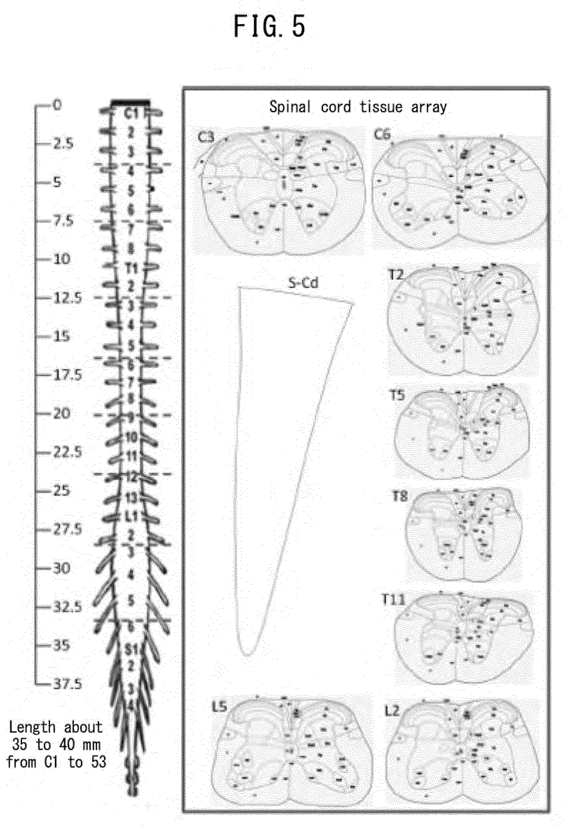

Fig. 5. Sectioning levels in tissue array of spinal cord. Drawings of spinal cord sections are taken from Allen Brain Atlas (http://mousespinal.brain-map.org/mageseries/showref.html).

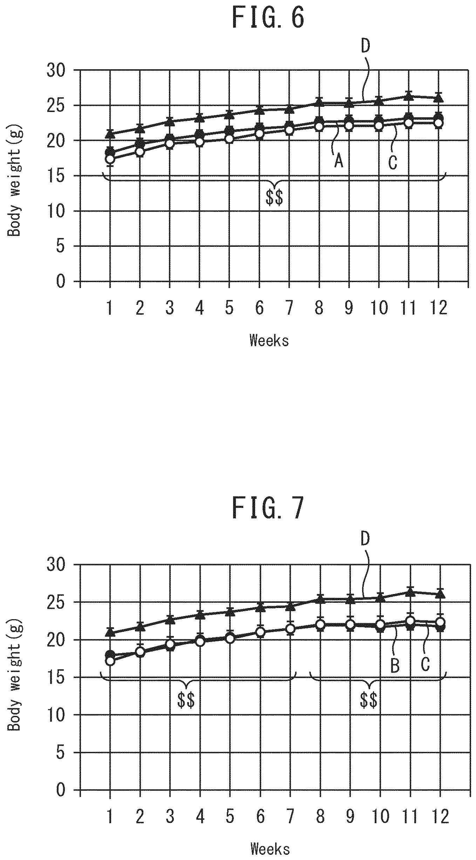

Fig. 6. Body weights: Graph represents the progress of body weights [g] per group (A, C and D) measured once a week over the whole treatment period. Each paint represents the mean ± SEM of all animals per group and week. Group C was compared to group A and D ($$: p<0.01 group D vs. group C, Repeated Measures ANOVA, Factor: group).
Fig. 7. Body weights: Graph represents the progress of body weights [g] per group (B, C and D) measured once a week ever the whole treatment period. Each point represents the mean ± SEM of all animals per group and week. Group C was compared to Group B and D ($$: p<0.01 group D vs. group C, Repeated Measures ANOVA, Factor: group).
Fig. 8. Hanging-wire: Graph represents the progress of wire suspension time [sec] per group (A, C and D) measured in week one and further biweekly over the whole treatment period. Each point represents the mean ± SEM of all animals per group and week. Group C was compared to group A and D (&; p<0.05 group C vs. group A, $$: p<0.01 group D vs. group C, Repeated Measures ANOVA, Factor: group).
Fig. 9. Hanging-wire: Graph represents the progress of time [sec] until falling off the wire per group (B, C and D) measured in week 1 and further biweekly over the whole treatment period. Each point represents the mean ± SEM of all animals per group and week. Group C was compared to group B and D ($$ p<0.01 Group D vs. Group C, Repeated Measure ANOVA, Factor: group).
Fig. 10. RotaRod: Graph represents the mean latency to fall [sec] for trial 1, 2, 3 and mean per group at Baseline testing and in week 12. Group C was compared to group D, and group A to B were compared to group C (##: p<0.01, t-test). Data are displayed as a bar graph of mean ± SEM of all animals per group.
Fig. 11. Clasping: Graph represents the mean clasping score [n] per group at Baseline testing and in week 12. Group C was compared to group D, and group A to B were compared to group C (##: p<0.01. t-test). Data are displayed as a bar graph of mean ± SEM of all animals per group.
Fig. 12. hTDP-43: Graphs present the means of immunofluorescent signal measured within the ROIs of 1 spinal cord tissue array per mouse (n = 8 per group). Each value represents Mean ± SEM. *: p<0.05, vs. Group C (Dunnett's test); #: p<0.05, ##: p<0.01, vs. Group D (t test); $$: p<0.01, vs. Group C (Dunnett's test followed by 2-way ANOVA; Factor: Group); %%: p<0.01, vs. Group D (2-way ANOVA; Factor: Group).
Fig. 13. GFAP: Graphs present the means of immunofluorescent signal measured within the ROIs of 1 spinal cord tissue array per mouse (n = 8 per group). Each value represents Mean ± SEM. *: p<0.05, **: p<0.01, vs. Group C (Dunnett's test); #: p<0.05, ##: p<0.01, vs. Group D (t test); $$: p<0.01, vs. Group C (Dunnett's test followed by 2-way ANOVA; Factor: Group); %: p<0.05, %%: p<0.01, vs. Group D (2-way ANOVA; Factor: Group).
Fig. 14. MAP2: Graphs present the means of immunofluorescent signal measured within the ROIs of 1 spinal cord tissue array per mouse (n = 8 per group). Each value represents Mean ± SEM. $: p<0.05, vs. Group C (Dunnett's test followed by 2-way ANOVA; Factor: Group); %: p<0.05, %%: p<0.01, vs. Group D (2-way ANOVA; Factor: Group).
Fig. 15. Iba1: Graphs present the means of immunofluorescent signal measured within the ROIs of 1 spinal cord tissue array per mouse (n = 8 per group). Each value represents Mean ± SEM. #: p<0.05, ##: p<0.01, vs. Group D (t test); $: p<0.05, vs. Group C (Dunnett's test followed by 2-way ANOVA; Factor: Group); %: p<0.05, %%: p<0.01, vs. Group D (2-way ANOVA; Factor: Group).
Fig. 16. hTDP-43: Graphs present the means of immunofluorescent signal measured within the ROI on 5 brain sections per mouse (n = 8 per group). Each value represents Mean ± SEM. ##: p<0.01 vs. Group D (t test).
Fig. 17. GFAP: Graphs present the means of immunofluorescent signal measured within the ROI on 5 brain sections per mouse (n = 8 per group). Each value represents Mean ± SEM. ##: p<0.01 vs. Group D (t test).
Fig. 18. Iba1: Graphs present the means of immunofluorescent signal measured within the ROI on 5 brain sections per mouse (n = 8 per group). Each value represents Mean ± SEM. #: p<0.05, ##: p<0.01 vs. Group D (t test).
Fig. 19 NeuN: Graphs present the means of immunofluorescent signal measured within the ROI on 5 brain sections per mouse (n = 8 per group). Each value represents Mean ± SEM. #: p<0.05, ##: p<0.01 vs. Group D (t test).
Fig. 20 Western Blot: Graphs present the means of TDP-43 signals (AU) or calculated ratio (TDP-43 normalized to Tubulin isotype III) of all animals (Hippocampus: n=15 per group, Spinal Cord: n= 7 per group). Each value represents Mean ± SEM. $: p<0.05, vs. Group C (Dunnett's test followed by 2-way ANOVA; Factor: Group); %: p<0.05, %%: p<0.01, vs. Group D (2-way ANOVA; Factor: Group). ##: p<0.01, vs. Group D (t test).

### DESCRIPTION OF EMBODIMENTS

The present invention relates to a cell preparation and/or a pharmaceutical composition for treating, preventing, alleviating and/or delaying the onset of motor neuron diseases (MNDs) in subjects, containing SSEA-3 positive pluripotent stem cells (Muse cells), and to a treatment method of MNDs using the cell preparation, etc. The present invention will now be explained in greater detail.

Abbreviations used herein are detailed below. Incidentally, abbreviations generally used in the art shall follow the conventions.

### Abbreviations used:

Ang1: angiopoietin-1
ANOVA: analysis of variance
BW: body weight
BRET: bioluminescence resonance energy transfer
EGF: epidermal growth factor
FACS: fluorescence-activated cell sorting
GFAP: glial fibrillary acidic protein
GFP: green fluorescence protein
HBSS: Hank's Balanced Salt Solution
HE: hematoxylin and eosin
HGF: hepatocyte growth factor
Iba1: ionized calcium-binding adapter molecule 1
IGF-1: insulin-like growth factor 1
i.t.: intrathecal
i.v.: intravenous
MAP2: microtubule-associated protein 2
MSC: mesenchymal stem cell
NMJ: neuromuscular junction
NeuN: neuronal nuclei
PGE2: prostaglandin E2
SOD1: superoxide dismutase 1
S1P: sphingosine-1-phosphate
S1PR2: sphingosine-1-phosphate receptor 2
TDP-43: TAR DNA binding protein 43
Tg: transgenic
VAChT: vesicular acetylcholine transporter
VEGF: vascular endothelial growth factor
WT: wild type

### 1. Applicable disease

The present invention can treat, prevent, ameliorate and/or delay the onset of motor neuron diseases (hereinafter sometimes abbreviated simply as " MNDs"), such as myotrophic lateral sclerosis (ALS), primary lateral sclerosis (PLS), treatment of motor neuron diseases (hereinafter sometimes abbreviated simply as " MNDs") such as spinal muscular atrophy (SMA), progressive muscular atrophy (PMA), or spinobulbar muscular atrophy (SBMA), by use of a cell preparation or a pharmaceutical composition containing SSEA-3-positive pluripotent stem cells (Muse cells).

Furthermore, the present invention can treat, prevent, ameliorate and/or delay the onset of frontotemporal lobar degeneration (FTLD) with TDP-43 accumulates in nerve cells (FTLD-TDP), by use of a cell preparation or a pharmaceutical composition containing SSEA-3-positive pluripotent stem cells (Muse cells).

As used herein, the term "motor neuron diseases (MNDs)" refers to neurological disorders that selectively destroys motor neurons. In general, based on the diagnostic criteria of "ICD-10 (International Statistical Classification of Disease and Related Health Problems 10th revision)" of the World Health Organization (WHO), motor neuron diseases (also referred to as a motor neurodegenerative disease) is classified into Chapter VI (G) "Diseases of the Nervous System" [G12: Spinal Muscular Atrophy and Related Syndromes] Syndrome)], preferably [G12.2: Motor Neuron Disease]. Examples of MNDs typically include amyotrophic lateral sclerosis (including familial amyotrophic lateral sclerosis), primary lateral sclerosis, spinal muscular atrophy (distal spinal muscular atrophy, familial spinal muscular atrophy, scapulofibular muscular atrophy), progressive muscular atrophy (juvenile progressive muscular atrophy, childhood progressive muscular atrophy, infantile progressive bulbar palsy, Spinal cord progressive muscular atrophy, etc.), spinal and bulbar muscular atrophy, Werdnig-Hoffmann disease, diffuse atrophic paralysis, motor neuron disease, pseudobulbar palsy, bulbar palsy, juvenile unilateral upper extremity muscular atrophy, progressive bulbar palsy, traumatic bulbar palsy, and cervical muscular atrophy.

MNDs, neurological disorders that selectively destroy and/or degenerate motor neurons, can lead to death. Motor neurons (including primary (upper) and secondary (lower) motor neurons) act on voluntary muscles to stimulate them to contract. Primary motor neurons originate in the cerebral cortex, send fibers through the brainstem and spinal cord, and are involved in the control of secondary motor neurons. Secondary motor neurons are located in the brainstem and spinal cord and send fibers to muscles. Secondary motor neuron diseases are diseases involving degeneration of secondary motor neurons. When a secondary motor neuron degenerates, the muscle fibers that it normally activates are cut and stop contracting, causing muscle weakness and hyporeflexia. Loss of neurons of either type leads to weakness, and muscle atrophy (muscle wasting) and painless weakness are clinical features of MNDs.

"Amyotrophic lateral sclerosis (ALS)" is fatal motor neuron diseases characterized by selective and progressive loss of motor neurons in the spinal cord, brainstem, and cerebral cortex. It typically leads to progressive muscle weakness and neuromuscular respiratory failure. About 10% of ALS are associated with point mutations in the gene encoding the Cu/Zn superoxide dismutase 1 enzyme (SOD1). Herein, a mutant SOD 1 (G93A) transgenic mouse model was used to study the therapeutic efficacy of the cell preparations of the present invention (see Example 1). On the other hand, it is known that abnormal accumulation of phosphorylated or ubiquitinated TDP-43 in neurons is involved in the onset of ALS. Abnormalities in the gene coding TDP-43 and abnormalities in the TDP-43 degradation mechanism have been reported, and are found in some familial ALS (ALS 10, with mutations in the TARDBP gene) and about 90% of sporadic ALS. In the present specification, an ALS mouse model overexpressing TDP-43 was generated to examine the therapeutic efficacy of the cell preparation of the present invention (see Example 2).

"Primary lateral sclerosis (PLS)" is a disease of unknown cause in which only primary (upper) motor neurons are selectively and progressively damaged, while secondary (lower) motor neurons are preserved. PLS can be difficult to distinguish from ALS, which fronts primary motor neuron damage, and is sometimes associated with frontotemporal lobar degeneration (FTLD).

"Spinal muscular atrophy (SMA)" is a neurogenic muscular atrophy caused by lesions of the motor neurons of the spinal cord (anterior horn cells of the spinal cord). Muscle weakness and atrophy of the trunk and extremities are progressive. SMA is classified into type I: severe (also known as Werdnig-Hoffmann disease), type II: intermediate (also known as Dubovitz disease), and type III: mild (also known as Kugelberg-Welander disease) (the types develop in childhood); and type which develops in adulthood.

"Progressive muscular atrophy (PMA)" is a disease that causes progressive weakness and atrophy of the skeletal muscles of the limbs and trunk. Among those with causes in the muscle itself are muscular dystrophy and atrophic myotonia. Muscular dystrophy is defined as a disease based on gene mutation whose main pathology is skeletal muscle necrosis/regeneration.

Spinal and bulbar muscular atrophy (SBMA) is a neurological disease caused by the gradual loss of nerves (secondary motor neurons) that move muscles in the brainstem and spinal cord, which are parts of the brain, which affects only adult males. The cause of spinal and bulbar muscular atrophy is known to be an abnormality in the "androgen receptor gene" on the sex-determining X chromosome.

"Frontotemporal lobar degeneration (FTLD)" is characterized by causing neurodegeneration due to degeneration and/or loss of nerve cells mainly in the frontal lobe and temporal lobe of the cerebrum, and leading to slowly progressive personality changes, behavioral abnormalities, aphasia, cognitive dysfunction, and movement disorders. In FTLD, accumulation of protein inclusion bodies is observed in nerve cells. Depending on the type of inclusion protein, FTLD is classified into FTLD-TDP (accumulation of TDP-43), FTLD-TAU (accumulation of TAU), FTLD-FUS (accumulation of FUS), FTLD-others (accumulation of other proteins), etc. FTLD-TDP accounts for about 45% of FTLD and is classified into types A to D based on histopathological features.

### 2. Cell preparation

### (1) Pluripotent stem cells (Muse cells)

The pluripotent stem cells to be used in the cell preparation of the present invention are typically cells whose existence in the human body was discovered by Prof. Dezawa, one of the present inventors, and which are named "Muse (Multilineage-differentiating Stress Enduring) cells". Muse cells can be obtained from bone marrow fluid and adipose tissue (Ogura, F., et al., Stem Cells Dev., Nov 20, 2013 (Epub) (published on Jan 17, 2014)) or from skin tissue such as dermal connective tissue, and they are widely dispersed throughout the connective tissue of various organs. The cells have the properties of both pluripotent stem cells and mesenchymal stem cells, and are identified as being double-positive for the cell surface markers "SSEA-3 (Stage-specific embryonic antigen-3)" and "CD105". Therefore, Muse cells or cell populations containing Muse cells, for example, can be isolated from body tissue using these antigen markers. The methods of separation and identification of Muse cells, and their features, are disclosed in detail in International Patent Publication No. WO2011/007900. Also, as reported by Wakao et al. (S. Wakao, et al., Proc. Natl. Acad. Sic. USA, Vol. 108, p. 9875-9880 (2011)), when mesenchymal cells are cultured from the bone marrow or skin and used as a parent population of Muse cells, all of the SSEA-3 positive cells are also CD105-positive. Consequently, when Muse cells are isolated from mesenchymal tissue of a body or cultured mesenchymal stem cells for the cell preparation of the present invention, the Muse cells may be used after purification with SSEA-3 alone as the antigen marker. Throughout the present specification, pluripotent stem cells (Muse cells) or a cell population containing Muse cells, isolated from mesenchymal tissue of a body or cultured mesenchymal tissue using SSEA-3 as the antigen marker, and which can be used in a cell preparation (including pharmaceutical composition) for treatment of motor neuron diseases, may be referred to simply as "SSEA-3 positive cells". Also throughout the present specification, "non-Muse cells" refers to cells that are present in mesenchymal tissue of a body or cultured mesenchymal tissue, and are the remainder of "SSEA-3 positive cells".

In brief, Muse cells or a cell population containing Muse cells can be isolated from body tissue (for example, mesenchymal tissue) using only antibody for the cell surface marker SSEA-3, or using both antibodies for SSEA-3 and CD105. The term "body" here means "mammalian body". According to the present invention, the "body" does not include a fertilized ovum or an embryo at a developmental stage before the blastocyst stage, but it does include an embryo at the developmental stage from the blastocyst stage onward, including the fetus or blastocyst. The mammal is not limited and may be a primate such as human or monkey, a rodent such as a mouse, rat, rabbit or guinea pig, or a cat, dog, sheep, pig, cow, horse, donkey, goat or ferret. The Muse cells to be used in the cell preparation of the present invention are clearly distinguished from embryonic stem cells (ES cells) or iPS cells based on separation from body tissue using a direct marker. The term "mesenchymal tissue" refers to tissue from the bone, synovial membrane, fat, blood, bone marrow, skeletal muscle, dermis, ligament, tendon, dental pulp, umbilical cord or umbilical cord blood, or tissues present in various organs. For example, the Muse cells may be obtained from the bone marrow or skin or adipose tissue. Preferably, mesenchymal tissue of a body is harvested and the Muse cells are isolated from the tissue and used. The separating means mentioned above may be used to separate Muse cells from cultured mesenchymal cells such as fibroblasts or bone marrow-derived MSCs. The Muse cells to be used for the cell preparation and pharmaceutical composition of the present invention may be either autologous or allogenic with respect to the recipient.

As mentioned above, Muse cells or a cell population containing Muse cells can be isolated from body tissue using SSEA-3 positivity, or double positivity for SSEA-3 and CD105, as indicators, but human adult skin is known to include various types of stem cells and progenitor cells. However, Muse cells are not identical to these cells. Such stem cells and progenitor cells include skin-derived precursors (SKP), neural crest stem cells (NCSC), melanoblasts (MB), perivascular cells (PC), endothelial precursor cells (EP) and adipose-derived stem cells (ADSC). Muse cells can be separated out as being "non-expressing" for the markers unique to these cells. More specifically, Muse cells can be separated by using non-expression for at least one, and for example, 2, 3, 4, 5, 6, 7, 8, 9, 10 or 11 among 11 markers selected from the group consisting of CD34 (EP and ADSC marker), CD117 (c-kit) (MB marker), CD146 (PC and ADSC marker), CD271 (NGFR) (NCSC marker), NG2 (PC marker), vWF (von Willebrand factor) (EP marker), Sox10 (NCSC marker), Snai1 (SKP marker), Slug (SKP marker), Tyrp1 (MB marker) and Dct (MB marker). As a non-limitative example, non-expression of CD117 and CD146 may be used as the indicator for separation, non-expression of CD117, CD146, NG2, CD34, vWF and CD271 may be used as the indicator, or non-expression of all of the aforementioned 11 markers may be used as the indicator for separation.

The Muse cells having the aforementioned features to be used for the cell preparation and pharmaceutical composition of the present invention may have at least one property selected from the group consisting of the following:
(i) low or no telomerase activity;
(ii) having the ability to differentiate into any of the three germ layers;
(iii) exhibiting non-tumorigenic proliferation; and
(iv) having self-renewal ability.

According to one aspect of the present invention, the Muse cells to be used for the cell preparation of the present invention have all of these properties. As regards (i), "low or no telomerase activity", this refers to low or non-detectable telomerase activity when using a TRAPEZE XL telomerase detection kit (Millipore), for example. "Low" telomerase activity is, for example, either telomerase activity on the same level as human fibroblasts, which are somatic cells, or telomerase activity of 1/5 and preferably no greater than 1/10 of that of HeLa cells. In regard to (ii), the Muse cells have the ability to differentiate into the three germ layers (endoderm, mesoderm and ectoderm) *in vitro* and *in vivo,* and by induction culturing *in vitro,* for example, they can differentiate into hepatocytes, neurons, skeletal muscle cells, smooth muscle cells, osteocytes or adipocytes. They may also exhibit the ability to differentiate into the three germ layers in the case of transplanting *in vivo* into the testes. They also have the ability to migrate and engraft onto damaged organs (heart, skin, spine, liver, muscle, etc.), by administration into the body via intravenous injection, and differentiate into cells that are compatible to the engrafted tissue. In regard to (iii), the Muse cells have the property of proliferating at a rate of about every 1.3 days in suspension culture, and growing in suspension culture from a single cell to form an embryoid-like cell cluster, slow down the growth at about 14 days; however, when the embryoid-like cell cluster is carried into adhesion culture, cell growth resumes and the proliferated cells spread out from the cell cluster. They also have the property of not generating teratomas at least for 6 months after transplantation into the testes. In regard to (iv), Muse cells have self-renewal (auto-replicating) ability. The term "self-renewal" means that cells in the embryoid-like cell cluster obtained by culturing a single Muse cell in suspension culture can be confirmed to differentiate into cells of all 3 germ layers, and also that when a single cell from the embryoid-like cell cluster is again carried into a suspension culture, it forms a next generation embryoid-like cell cluster, and reproduce differentiation into three germ layers as well as embryoid-like cell cluster in the suspension culture can be confirmed. Self-renewal may be observed once or as several repeated cycles.

In addition, a cell fraction containing Muse cells to be used in the cell preparation of the present invention may be a cell fraction having the SSEA-3 positive and CD105-positive pluripotent stem cells concentrated, obtained by a method of applying external stress treatment to mesenchymal tissue of a body or cultured mesenchymal cells, causing the cells other than the external stress-resistant cells to die, and recovering the surviving cells, the cell fraction having at least one and preferably all of the following properties.
(i) SSEA-3 positivity;
(ii) CD105-positivity;
(iii) low or no telomerase activity;
(iv) having the ability to differentiate into any of the three germ layers;
(v) exhibiting non-tumorigenic proliferation; and
(vi) having self-renewal ability.

The external stress may be any one or a combination of: protease treatment, culturing in a low oxygen concentration, culturing under low-phosphate conditions, culturing with low serum concentration, culturing under low nutritive conditions, culturing under exposure to heat shock, culturing at low temperature, freezing treatment, culturing in the presence of a hazardous substance, culturing in the presence of active oxygen, culturing under mechanical stimulation, culturing with agitating treatment, culturing with pressure treatment, or physical impact. For example, the treatment time with a protease is preferably a total of 0.5 to 36 hours to apply external stress to the cells. The protease concentration may be the concentration used when the cells adhering to a culture vessel are detached, when the cell aggregation is dispersed into individual cells, or when individual cells are recovered from tissue. The protease is preferably a serine protease, aspartic acid protease, cysteine protease, metalloprotease, glutamic acid protease or N-terminal threonine protease. The protease is also preferably trypsin, collagenase or dispase.

### (2) Preparation and use of cell preparation

Although not limited thereto, the cell preparation of the present invention may be obtained by suspending the Muse cells or a cell population containing Muse cells obtained by (1) above, in physiological saline or an appropriate buffer (for example, phosphate-buffered physiological saline). In this case, when the number of Muse cells isolated from autologous or allogenic tissue is low, the cells may be cultured before administration for growth until the prescribed cell density is obtained. As already reported (International Patent Publication No. WO2011/007900), Muse cells do not transform into tumorigenic cells, and therefore even if the cells recovered from body tissue are in undifferentiated form, they have low tumorigenicity and are safe. There are no particular restrictions on culturing of the recovered Muse cells, and it may be carried out in ordinary growth medium (for example, α-MEM containing 10% newborn calf serum). More specifically, referring to International Patent Publication No. WO2011/007900, suitable medium and additives (for example, antibiotics and serum) may be selected for culturing and growth of the Muse cells, and a solution containing the prescribed density of Muse cells may be prepared. When a cell preparation of the present invention is to be administered to a human patient, roughly several milliliters of bone marrow fluid may be harvested from human iliac bone, and for example, the bone marrow-derived MSCs may be cultured as adherent cells from the bone marrow fluid to increase them to a number of cells allowing separation of an effective therapeutic amount of Muse cells, after which the Muse cells may be separated out with SSEA-3 antigen marker as the indicator, and autologous or allogenic Muse cells prepared as a cell preparation. As an alternative example, Muse cells that have been separated using SSEA-3 antigen marker as the indicator, and the cells cultured to increase them to an effective therapeutic amount, may then be prepared as a cell preparation of autologous or allogenic Muse cells.

For use of the Muse cells in a cell preparation, dimethyl sulfoxide (DMSO) or serum albumin may be added to the cell preparation or pharmaceutical composition to protect the cells, and an antibiotic or the like may be added to prevent infiltration and growth of bacteria. In addition, other pharmaceutically acceptable components (for example, carriers, excipients, disintegrators, buffering agents, emulsifying agents, suspending agents, soothing agents, stabilizers, preservatives, antiseptic agents, physiological saline and the like), or cells or components other than Muse cells that are present among MSCs, may be added to the cell preparation or pharmaceutical composition. A person skilled in the art may add such factors and chemical agents to the cell preparation in appropriate concentrations. Thus, Muse cells can also be used as pharmaceutical compositions containing various additives.

The number of Muse cells contained in the cell preparation or pharmaceutical composition to be prepared may be appropriately adjusted so as to obtain the desired effect for treatment of motor neuron diseases (for example, increased muscle strength and suppression of progression of muscle atrophy), in consideration of the target gender, age and body weight, the state of the affected area, and the state of the cells to be used. The target individual includes, but is not limited to, mammals such as humans. In addition, the cell preparation of the present invention may be administered once, but may be administered multiple times (for example, 2 to 10 times or more) at appropriate intervals (for example, twice a day, once a day, twice a week, once a week, once every two weeks, once every three weeks, once every four weeks, once a month, once every two months, once every three months, once every six months, etc.) until the desired therapeutic effect is obtained. The timing of administration may be early, intermediate, or late onset as long as a therapeutic effect is obtained.

The therapeutically effective amount, while depending on the condition of the subject, may be, for example, a dose 1 × 10³ cells to 1 × 10¹¹ cells per individual, preferably 1 × 10⁴ cells to 1 × 10¹⁰ cells, more preferably 1 × 10⁵ cells to 1 × 10⁹ cells and the like. In addition, the cell preparation of the present invention may be administered directly to the brain (brainstem, cerebral cortex) or spinal cord, or may be administered intravenously, although it is not particularly limited. Moreover, from Examples, etc., intravenous administration is also a preferred aspect.

Human-derived Muse cells can be used in the cell preparation and pharmaceutical composition of the present invention. For subjects (e.g., mice and rats) who have a heterologous relationship with the Muse cells to be administered, an immunosuppressive agent (such as cyclosporin) may be administered either before or simultaneously with administration of the allogenic cells to suppress *in vivo* rejection of the heterogenous cells. According to the present invention, when the cell preparation and pharmaceutical composition of the present invention are applied to the treatment of motor neuron diseases, etc., such an immunosuppressive agent may or may not be used in combination. In addition, when the cell preparation and pharmaceutical composition of the present invention are administered to a patient, it is also a preferred aspect not to use an immunosuppressive agent in combination.

### 3. Therapeutic effect of Muse cells

In embodiments of the present invention, the cell preparations and pharmaceutical compositions of the present invention can treat, prevent, alleviate and/or delay the onset of motor neuron diseases (MNDs). More specifically, the present invention can restore muscle strength in the trunk and limbs or inhibit progression of muscle atrophy caused by the MNDs. Here, the term "treatment" refers to suppression or complete elimination of various symptoms caused by MNDs. In addition, the term "alleviation" means alleviation of various symptoms and inhibition of progression caused by MNDs, preferably alleviation of symptoms to the extent that daily life is not disturbed. The term "alleviation" can also include the meaning of "delaying symptoms."

In this specification, as shown in the Examples below, the effectiveness of the cell preparation or pharmaceutical composition of the present invention can be evaluated by using ALS model mutant SOD1 (G93A) Tg mice or TDP-43 Tg mice to measure the exercise capacity of TDP-43 Tg mice. Commonly used methods such as, but not limited to, hanging-wire test, RotaRod, clasping, grip strength test, and beam balance test, etc., can be employed as evaluation methods.

Hereinafter, the present invention is described in more detail by referenced to the Examples, but the present invention is not limited to the Examples.

### EXAMPLE

All animal experiments were approved by the Institutional Animal Care and Use Committee of Okayama University (OKU-2019289), and performed in accordance with the guidelines of Okayama University on animal experiments.

### Example 1

### Materials and Methods

### Animals and experimental groups

Transgenic (Tg) mice with the G93A human SOD1 mutation (G1H/+) were obtained from Jackson Laboratories (Bar Harbor, ME, USA) [2] and maintained as hemizygotes by mating Tg males with C57BL/6J females. Comparative experiment between intravenous (i.v.) and intrathecal (i.t.) injections used 3 animals for each group, Nano-lantern ex-vivo imaging used 2 animals and for the vehicle, MSCs and Muse cells, respectively. For evaluation of therapeutic efficacy by i.v.-injection, the number of animals used were 10 (5 males and 5 females) in the vehicle group, 9 (4 males and 5 females) in the MSC group, and 9 (5 males and 4 females) in the Muse cell group.

### Preparation of GFP-labeled MSC and Muse cells

GFP-labeled MSC was prepared by labelling human bone marrow-MSCs (Lonza, Basel, Switzerland) with lentivirus GFP as previously described [22]. GFP-labeled Muse cells were isolated as SSEA-3-positive cells from GFP-MSCs by fluorescence-activated cell sorting (FACS) as described [10, 11]. The above GFP-MSC and -Muse cells were frozen in Beissel freezing container (Nihon Freezer, Tokyo, Japan), and kept in the liquid nitrogen until use.

### In vivo comparative cell transplantation for injection route

The above GFP-labeled Muse cells (2.0 × 10⁵) in 250 µl of Hank's balanced salt solution (HBSS, pH 7.4) were injected into the tail vein for i.v. or into cisterna magna for intrathecal injection as previously described [23, 24]. At 7 days, all animals were sacrificed.

### Ex-vivo dynamics with Nano-lantern

Nano-lantern-labeling with bioluminescence resonance energy transfer (BRET) efficacy is the bright luminescent protein allowing detection even for small number of transplanted cells [25]. Human bone marrow-MSCs (Lonza) were labeled with Nano-lantern as previously described [25]. Nano-lantern-labeled Muse cells were isolated from Nano-lantern-labeled MSCs as SSEA-3-positive cells by FACS. For ex-vivo dynamics of i.v.-injection, vehicle (HBSS), Nano-lantern-labeled-MSC and -Muse cells (1.0 × 10⁵ cells/250 µl) were intravenously injected at 14 weeks old. After 7 days, animals were sacrificed under deep anesthesia and analyzed as described previously [26].

### Evaluation of therapeutic efficacy

Vehicle, GFP- MSC and GFP- Muse cells (both 5.0 × 10⁴ cells/250 µl) were injected into the tail vein of each animal over one min. Because our and other groups confirmed that G93A Tg mice were reported to exhibit early onset at around 56 days of age [27], we decided to start cell administration at 56 days of age, and continue the administration once in a week until 119 days (total 10 injections) with the same number of the cells. An immunosuppressive compound, cyclosporine A (10 mg/kg/day, Novartis International, Basel, Switzerland), was also applied intraperitoneally (IP) to all animals immediately after administration in the vehicle, MSC, or Muse groups every other day until sacrifice. Survival was checked every day, and body weight (BW) and the RotaRod score were measured twice a week from 56 days of age. Rotarod test and wheel-running activity were done according to our previous method [24, 28]. Hanging-wire test was evaluated once a week as a measure of muscular strength and coordination as previously reported [29]. Muscle strength of lower limb was measured once a week by using precision spring scale (Ooba Keiki Co., Tokyo, Japan).

### Immunohistological analysis

The mouse was euthanized when it became unable to stand up within 15 seconds after it fell to one side, and was recorded as the time of death. Each animal was deeply anesthetized by intraperitoneal injection of pentobarbital (20 mg/kg), and then subjected to sampling as previously described [26]. Primary antibodies used were: goat anti-GFP antibody (1:500, Abcam, Cambridge, UK); rabbit anti-GFP antibody (1:500, MBL, Woburn, USA); rabbit anti-Iba1 antibody (1:500, Wako, Osaka, Japan); mouse anti-betaIII tubulin (Tuj1) antibody (1:100, Santa Cruz Biotechnology); rabbit anti-glial fibrillary acidic protein (GFAP) antibody (1:500, Dako, Glostrup, Denmark); mouse anti-NeuN antibody (1:100, Millipore, MA, U.S.A.); goat anti-vesicular acetylcholine transporter (VAChT) antibody (1:200, Thermo Scientific). Secondary antibodies used were either anti-goat, -rabbit or -mouse IgG conjugated with Alexa 488 or 546 (1:500, Alexa Fluor^{™}, Invitrogen, Carlsbad, CA, USA). Alpha-Bungarotoxin conjugated with Alexa 594 (1:500, Millipore) was also used to detect acetylcholine receptors.

For cell-type-marker/GFP double-labeling, 5-6 areas from pia-mater to anterior horn were randomly selected and were analyzed. Nissl-stained motor neurons in L4-5 were counted using 5 transverse sections from each lumbar cord [30]. Cells larger than 20 µm with clear nucleoli in both ventral horns below a lateral line across the spinal cord from the central canal were counted as motor neurons [31]. For denervation, about 100 neuromuscular junctions (NMJ) from each mouse were analyzed. For myofiber size, about 180 myofibers from 3 hematoxylin and eosin (HE) stained tibialis anterior muscle sections per mouse were analyzed by an investigator blinded to the treatment conditions.

### Statistical Analyses

The investigators were blinded to the experimental group during data collection and analysis. The data that support the findings of this study are available from the corresponding author upon reasonable request. Data were analyzed in SPSS version 22.0.0.0 (IBM Corp., Armonk, New York, USA) and expressed as means ± SD. Therapeutic efficacy was evaluated by non-repeated measures analysis of variance (ANOVA) and the Dunnett't test. Immunohistological data was analyzed by using Kruskall-Wallis, followed by Mann-Whitney U-test with Bonferroni correction. In all statistical analyses, significance was assumed at p<0.05.

### Results

For determining administration route, homing of GFP-Muse cells was compared between i.v.- and i.t.-injections by immunohistological analysis of G93A mice spinal cord at 7 days. The pilot study demonstrated that the number of GFP-Muse cells was consistently low or neglectable in the cervical, thoracic and lumbar spinal cord in the i.t.-injection group, whereas that was higher in the cervical and lumbar spinal cord of the i.v.-injection group compared to the i.t.-injection group, and those GFP-Muse cells mainly located at pia-mater and the underneath white matter. GFP-Muse cells were scarcely detected in the thoracic spinal cord even by i.v.-injection (Table 1, Fig. 1a, b). Consequently, i.v.-injection was chosen as the administration route in the following experiments.

### [Table 1]

**Table 1. The number of GFP-labeled Muse cells detected in spinal cords**

| (in vivo comparative experiment between i.v. and i.t.) | | | | | | |
|---|---|---|---|---|---|---|
| | IV (n = 3) | | | IT (n = 2) | | |
| | animal No. | pia mater-white matter | ventral horn | animal No. | pia mater-white matter | ventral horn |
| Cervical | IV-① | + ∼ ++ | - | IT-① | - ∼ + | - |
| | IV-② | + ∼ +++ | + ∼ ++ | | | |
| | | | | IT-② | - ∼ + | - |
| | IV-③ | - | - | | | |
| Thoracic | IV-① | - | - | IT-① | - | - |
| | IV-② | - | - | | | |
| | | | | IT-② | - ∼ ± | - |
| | IV-③ | - ∼ ± | - | | | |
| Lumber | IV-① | + ∼ +++ | - | IT-① | - | - |
| | IV-② | - | - | | | |
| | | | | IT-② | - | - |
| | IV-③ | - | - | | | |

| | | | | | | |
|---|---|---|---|---|---|---|
| -, no GFP-positive cell; +, 1-4 /each section; ++, 5-9 /each section; +++, >10/ each section | | | | | | |

In order to examine the in vivo dynamics of MSCs and Muse cells after i.v., Nano-lantern-labeled cells were used. In the MSC group, intense signal was detected in the lung and a trace signal in the femur bone while not in other organs including the brain, cervical and lumbar spinal cord at day 7. In the Muse cell group, the signal was detected in the cervical and lumbar spinal cord (Fig. 1c, top right) as well as in the lung, while not in the brain (Fig. 1c, middle right). The signal in the femur bone was higher in the Muse cell group than in the MSC group. Immunohistological analysis confirmed the presence of Nano-lantern-Muse cells in the pulmonary vessel lumen and the bone marrow (Fig. 1d). The signal was consistently under detection limit in all organs inspected in the vehicle group (Fig. 1c).

The mean survival time showed no significant difference among the three groups (vehicle; 144.4 ± 8.0 days, MSCs; 143.9 ± 6.9 days, Muse cells; 142.6 ± 6.7 days). Body weight also didn't show any statistical difference among the three groups for the entire period (Fig. 2a). In contrast, RotaRod test showed an alleviation in the Muse group at 67 and 70 days with statistical significance to the vehicle groups (Fig. 2b). In addition, both hanging-wire score at 84, 112, and 133 days and muscle strength of lower limb at 126 and 140 days were also significantly improved only in the Muse cell group compared to the vehicle group (Fig. 2c, d).

In the lumber spinal cord, GFP-positive cell was undetectable in the vehicle group (Fig. 2e), whereas a small number of GFP-positive cells were observed in the MSC group (Fig. 2f). In the Muse cell group, GFP-positive cells were recognized at the spinal pia-mater (Fig. 2g, solid square), the underneath white matter and the ventral horn (Fig. 2g, dotted square, 2h), and 85.7% (180 out of 210 GFP-positive cells) of those cells co-expressed astrocytic marker GFAP locating both in the pia-mater (Fig. 2i) and ventral horn (Fig. 2j). The remainder of GFP-positive cells (14.3%) did not show any positivity for microglial marker Iba-1 (Fig. 2k, 0/120 GFP-positive cells), neuronal markers Tuj1 (Fig. 2l, 0/97 GFP-positive cells) nor NeuN (Fig. 2m, 0/109 GFP-positive cells), suggesting that the majority of i.v.-injected Muse cells spontaneously differentiated mainly into astrocyte-lineage cells after homing into the lumbar spinal cord.

When compared with the wild type (WT) group, the number of surviving motor neurons of the ventral horn was significantly low in the vehicle, MSC and Muse cell groups (WT, Fig. 3a, b, *p<0.05). However, the Muse group exhibited a higher number of motor neurons compared to the vehicle group with statistical significance (Fig. 3a, b, #p<0.05). The vehicle and MSC groups did not show statistical difference between them (Fig. 3a, b).

In the tibialis anterior muscle, the number of innervated synapses was significantly decreased in the vehicle, MSC and Muse cell groups compared with the WT (Fig. 3c, d, *p<0.05), which was again recovered in the Muse cell group compared to the vehicle group with statistical significance (Fig. 3c, d, #p<0.05). Myofiber size analysis demonstrated the sever neurogenic myofiber atrophy in the vehicle and MSC groups, which was improved in the Muse group with statistical significance to both the vehicle (*p<0.05) and MSC (#p<0.05) groups (Fig. 3e, f).

### Discussion

The present study is the first report to demonstrate that multiple i.v. administration of Muse cells improved clinical scores in the RotaRod, hanging-wire and muscle strength of lower limb in the ALS model G93A Tg mice. i.v.-injected Muse cells homed to the lumbar spinal cord, lung and bone (Fig. 1c, d). In the lumbar spinal cord, GFP-positive Muse cells predominantly expressed astroglial marker GFAP and exhibited glia-like morphology at the end-stage (154 days old, Fig. 2g-j). In addition, the number of surviving motor neurons in the lumber spinal cord was higher compared to the vehicle group with statistical significance (Fig. 3a, b), which might have led to the alleviation of both the denervation and myofiber atrophy in the lower limb muscle (Fig. 3c-f).

In ALS model mice, i.v.-injection was superior to i.t.-injection in terms of delivering Muse cells to the critical therapeutic target, namely spinal cord, (Table 1, Fig. 1a, b). For ALS patients, i.v.-administration has greater advantages over i.t.-administration because of easy accessibility, less-invasiveness and less-burden. These may allow repeated administration of Muse cells to ALS patients. Recent studies demonstrated that Muse cells that express sphingosine-1-phosphate receptor 2 (S1PR2) can specifically home to damaged site by sensing sphingosine-1-phosphate (S 1P) produced by damaged cells ¹⁸. S 1P is a general damage signal common to all organs because it is produced by phosphorylation of sphingosine, one of the components of the cell membrane. Therefore, Muse cells could home to the spinal cord of ALS mice by i.v.-injection.

Nano-lantern imaging demonstrated the stronger signal of Muse cells in the femur bone rather than MSC (Fig. 1c). Bone marrow abnormality was reported in ALS [32]. Therefore, Muse cells selectively and actively accumulated to the femur bone marrow as well as to the spinal cord by lesion-dependent manner, in contrast the passive entrapment in the lung capillaries. Another interesting point is that i.v.-injected Muse cells migrated to spinal pia-mater, the underneath white matter, and the ventral horn (Fig. 2g-j), suggesting homing of Muse cells into the spinal cord via pial perforating arteries. On the other hand, i.t.-injected Muse cells were scarcely detected in the spinal cord (Fig. 1a, b). These results suggested that homing factors for Muse cells were not mainly paracrined to the spinal subarachnoid space, but were endocrined to the circulating system [32-35].

In animal disease models, i.v.-injected Muse cells spontaneously differentiated into tissue-constituting cells after specific homing to damaged tissues; i.e., they differentiated into neuronal cells and oligodendrocytes in a stroke model [16]. In contrast to stroke, ALS is chronic and progressive and the disease state is completely different from stroke, i.v.-injected Muse cells differentiated not into neuronal-linage but mainly into astroglial-lineage in the spinal cord of ALS mice. Reactive astrocyte is the important therapeutic target of ALS [36]. Muse cells produce various neurotrophic factors including hepatocyte growth factor (HGF), vascular endothelial growth factor (VEGF), insulin-like growth factor 1 (IGF-1), epidermal growth factor (EGF), prostaglandin E2 (PGE2) and angiopietin-1(Ang1) [17-19]. Therefore they might have supplied beneficial factors to motor neurons and astrocytes, preventing myofiber atrophy in ALS model. In addition, clinical trials for acute myocardial infarction, stroke, spinal cord injury, epidermolysis bullosa and neonatal cerebral palsy are currently being conducted all by i.v.-drip of donor Muse cells without HLA-matching or long-term immunosuppressive drugs. In the clinical trial of acute myocardial infarction, safety and remarkable cardiac function recovery were reported [21].

Overall, the present study successfully achieved, for the first time, that systemic administration of Muse cells showed a significant clinical benefit for the ALS mice model, in which i.v.-injected Muse cells preferentially migrated to the spinal cords, supplied astroglia, supported motor neuron survival and suppressed myofiber atrophy. Muse cells can be a promising cell resource for treatment of ALS patients.

### Example 2

### 1. Study Aim

The aim of the study was to evaluate the effect of Muse cells on motor function in TDP-43 Tg mice.

### 2. Test System and Justification of the Test System

Amyotrophic lateral sclerosis (ALS) is a neurodegenerative disease characterized by degeneration of the motor neurons in the brain and the spinal cord. There is evidence that an ubiquitinated protein called human transactive response (TAR) DNA binding protein 43 kDa (TDP-43) plays a central role in the etiology of ALS. TDP-43 Tg mice are a suitable model to study TDP-43 production and deposit in the cells. Imitating the clinical features of ALS, this model is useful to test the effects of drugs against ALS. For the proposed study mice overexpressing human wild-type TDP-43 (hTDP-43) under control of a neuronal murine Thy-1 promoter will be used. In homozygous mice, the human TDP-43 protein is 3.8-fold higher expressed than the endogenous protein in non-transgenic littermates.

### 3. Preparation of Human Muse Cells

Human Muse cells were obtained and identified according to the method described in WO2011/007900. More specifically, Muse cells were obtained by expansive enrichment culture of mesenchymal stem cells under stress conditions. Animals received an amount of 5 mL/kg of the 2 × 10⁶ viable cells/ml working solution.

### 4. Vehicle

Hank's Balanced Salt Solution, no calcium, no magnesium, no phenol red (HBSS; Gibco 14175-046) was used as vehicle.

### 5. Animal Management

### 5-1. Housing

Animals were housed in individual ventilated cages on standardized rodent bedding supplied by Rettenmaier. A maximum at 5 animals at the same group were housed in one cage. The room temperature was maintained at approximately 21°C and the relative humidity was maintained between 40 to 70%. Animals were housed under a constant light/dark-cycle (12 hr / 12 hr). Dried, pelleted standard rodent chow (Altromin) as well as normal tap water was available to the animals ad libitum.

### 5-2. Environmental Enrichment

From the beginning of the first treatment, all animals were domesticated, with a mouse igloo and a FAST Trac (from PLEXX) as environmental enrichment.

### 5-3. Identification

Animals were numbered consecutively by classical ear punching. Each cage was identified by a colored card indicating the study number, sex, the individual registration numbers (IRN) of the animals, date of birth, as well as the genotyping date and the treatment group allocation.

The genotype of each animal was determined by polymerase chain reaction specific for the transgenic construct. Each mouse was genotyped prior to study start using DNA isolated from ear tissue collected during ear punching for animal identification.

### 5-4. Group Allocation

Only animals in apparently good health condition were included to the study. All animals were randomly assigned (by considering a well distributed mean body weight and sex over all groups and cohorts) to cohort comprising animals at all treatment groups. The number of animals in the first cohort was limited to ensure same age and uniform handling.

### 5-5. Health Status and Cage-side Observations

Before assignment to the study, the health status of each individual animal was evaluated. During the study, any notable observations from outside the cage were recorded, and further actions (e.g. euthanasia) were decide on further actions.

Body weights at all animals were recorded once a week. If the animals showed heavy motor dysfunctions, and were no longer able to reach food/water bottle, the mice were allowed access to food pellets and water on the bottom of the cage. Also drinking bottles with an extra-long cap were be used.

### 5-6. Handling of Prematurely Dead Animals

Animal that died prematurely (IRN 5921) was subjected to necropsy. The death rate was not evaluated because no other animals were euthanized or died prematurely.

### 6. Material and Methods

A number of 45 homozygous TDP-43 Tg mice, allocated to 3 groups with 15 animals each, and a group of 15 non-transgenic littermates at an age of 8 weeks were used for this study. Animals received either Muse cells or vehicle via intravenous (i.v.) application twice.

As a baseline test all animals were subjected once to Modified Irwin Test, including reflex tests and hanging-wire test. To evaluate motor coordination hanging-wire was performed every other week during the study (total of 6 times), RotaRod twice (baseline and at study end) and Clasping behavior twice (baseline and at study end).

At study end all animals were euthanized by intraperitoneal injection of pentobarbital. After transcardial perfusion with saline, brains were removed and separated into left and right hemibrains. The left hemibrain was divided into hippocampus and rest, and frozen on dry ice for protein expression analysis. The right hemibrain was post fixed in 4% PFA, embedded and frozen in cryomolds for immunohistological evaluations. Also, spinal cords were collected, n=7 were frozen on dry ice, the remaining n=8 spinal cords were post fixed and processed for immunohistological evaluations.

### 6-1. Animals

**[Table 2]**

| | |
|---|---|
| Mouse line: | TDP-43 TAR6/6 |
| Breeder: | QPS Austria |
| Age at start: | 8 Weeks ± 1 week |
| Sex: | Mixed |
| Number of animals: | 45 horn and 15 ntg |
| Remarks: | N/A |

### 6-2. Treatment

A number of 45 homozygous TDP-43 Tg mice, allocated to 3 groups with 15 animals each, and a group of 15 non-transgenic littermates at an age of 8 weeks were used as non-disease control for this study. In the 12-week study each animal received two intravenous (i.v.) injections of either Muse cells and vehicle, vehicle and Muse cells or vehicle and vehicle in weeks 1 and 8, respectively. That means Muse cells were given once only, either in week 1 (group A) or week 8 (group B). The following groups were used:

**[Table 3]**

| Group | N | Genotype | Dose article | |
|---|---|---|---|---|
| | | | week 1 | week 8 |
| A | 15 | horn | Muse cell ^{a)} | Vehicle ^{b)} |
| B | 15 | horn | Vehicle ^{b)} | Muse cell ^{a)} |
| C | 15 | horn | Vehicle ^{b)} | Vehicle ^{b)} |
| D | 15 | ntg | Vehicle ^{b)} | Vehicle ^{b)} |

| | | | | |
|---|---|---|---|---|
| ^{a)} 1 × 10⁷ cells/kg, i.v. ^{b)} HBSS i.v. | | | | |

### 6-3. Behavior

As a baseline test all animals were subjected once to Modified Irwin Test, including reflex tests and hanging-wire test. To evaluate motor coordination hanging-wire was performed every other week during the study (total of 6 times), RotaRod twice (baseline and at study end) and Clasping behavior twice (baseline and at study end).

Testing was performed in a randomized order.

### (1) Hanging-Wire Test

The hanging-wire Test assesses neuromuscular abnormalities measures of motor strength and was performed every other week during the study (starting in treatment week 2) and as part of the Irwin test (as baseline behavior).

For this test a wire cage lid was used where duct tape was placed around the perimeter to prevent the mouse from walking off the edge. The animal was placed on the top of the cage lid. The lid was lightly shaken three times to force the mouse to grip the wires and then it was turned upside down. The lid was held at a height of approximately 55-60 cm above a soft underlay, high enough to prevent the mouse from jumping down, but not high enough to cause harm in the event of a fall. The latency to fall down was quantified. A 300-secend cut-off time was used. A normal mouse can hang upside down for several minutes.

### (2) RotaRod

The RotaRod test was performed twice at study start (baseline) and at study end.

This test assesses motor coordination by placing the animals on a rotating rod (four-lane-Rota Rod; Ugo Basile) that ran at a constant or an accelerating speed. If a mouse lost its balance and fell onto an underlying platform, the red automatically stopped and recorded a measure of the latency to fall as well as the speed at fall.

Prior to the first test session, mice were habituated to the testing system, until they were able to stay on the rod at a constant speed of 2 rpm for approximately one minute. During testing, a single animal was exposed to the apparatus three times for a 180 sec trial. The initial speed increased from 2 to 20 rpm over an accelerating time of 180 seconds. If the mice fell, the session was over and the Ugo Basile Program stepped the timer.

### (3) Clasping Test

Clasping behavior was evaluated twice at study start (baseline) and at study end.

4 points: Full extension of hind legs away from lateral midline when mouse is suspended by its tail, and mouse can hold this for 2 seconds, suspended 2-3 times for a few seconds, animals should show movement with both hind legs during suspension at least two times.

3 points: Collapse or partial collapse of leg extension towards lateral midline (weakness) or trembling of hind legs during suspension.

2 points: Toes curl under at least twice during walking of 12 inches (30 cm), or at any part of foot is dragging along cage bottom/table.

1 point: Rigid paralysis or minimal joint movement, foot not for forward motion.

0 points: Meuse cannot right itself within (15-) 30 seconds from either side.

### 6-4. Tissue Sampling and Processing

At the end of the study all animals were euthanized by Pentobarbital injection (600 mg/kg) hemi brains and spinal cords were collected.

### (1) Perfusion

Mice were transcardially perfused with 0.9% saline. To this end, a 23-gauge needle connected to a bottle with 0.9% saline was inserted into the left ventricle. The right ventricle was opened with scissors. A constant pressure of 100 to 120 mm Hg was maintained on the perfusion solution by connecting the solution bottle to a manometer controlled air compressor. Perfusion was continued until the liver had turned pale and only perfusion solution instead of blood was exiting the right ventricle.

### (2) Brain sampling

After perfusion the skull was opened and afterwards the brain was removed carefully and hemisected on a cooled surface. The right hemi brain was fixed by immersion in freshly prepared 4% paraformaldehyde/PB (pH=7.4) for 2 hr at room temperature. The left hemibrain was further separated into hippocampus and rest brain. All parts were weighed, snap frozen on dry ice and stored at -80°C.

### (3) Spinal cord sampling

### Immunohistology (n =8 animals per group)

For immunohistological analysis the spinal column was dissected from the animal and as much of the muscle tissue was removed to facilitate immersion fixation afterwards. The whole spinal column including the cervical, thoracic and lumbar part was transferred into a 15 mL tube, containing freshly prepared 4% paraformaldehyde/PB, in an upright position for fixation. Spinal columns were fixated far 24 hr at 4°C.

### Protein expression analysis n=7 animals per group)

For protein expression analysis by Western blotting, the spinal column was dissected from the animal and the spinal cord was removed, weighed, snap frozen on dry ice and stored at -80°C.

### 6-5. Immunohistological Analysis

### (1) Tissue Preparation

Right hemi brains (n=15/group) were fixed by immersion in freshly prepared 4% paraformaldehyde in phosphate buffer (PB; pH 7.4) for 2 hr at room temperature. The samples were then transferred to 15% sucrose-PBS solution over night for cryoprotection. Afterwards, tissue blocks were trimmed as needed, were transferred to cryomolds and embedded in OCT medium, and were then snap-frozen in dry ice-cooled liquid isopentane and stored in an ultra deep freezer (set at -80°C target temperature) until sectioning.

Entire spinal columns (n=8/group) were fixed by immersion in freshly prepared 4% paraformaldehyde in PB (pH 7.4) for 24 hr at 4°C. On the next day the spinal cord was dissected from the fixated spinal column and transferred to 15% sucrose-PBS solution over night for cryoprotection. Afterwards, tissue blocks were cut into 8 pieces which were arranged as tissue array in a cryomold and embedded in OCT medium. The samples were then snap-frozen in dry ice-cooled liquid isopentane and stored in an ultra deep freezer (set at -80°C target temperature) until sectioning.

### (2) Sectioning

### Right hemi brains (n=8/group)

Five cryosections from 12 medio-lateral levels (60 sections in total) were sagittally cut at 10 µm thickness on a Leica cryotome. The next 23 sections per level were discarded. Sectioning levels were chosen according to the brain atlas of Paxinos and Franklin ("The Mouse Brain in Stereotaxic Coordinates", the 2nd edition, 2001). Collection of sections started at a level about 0.2 mm lateral from midline and was conducted through the hemisphere, in order to ensure systematic random sampling through the target regions (Fig. 4). Sections have been stored in the deep freezer (target temperature set at -20°C).

### Spinal cord tissue array (n=8/group)

Five cryosections from spinal cord tissue array were cut at 10 µm thickness on a Leica cryotome. The next 15 sections per level were discarded. This collection scheme was repeated for 5 levels (resulting in 30 sections in total). This resulted in collecting sections from cervical, thoracic, lumbar, and sacral segments and ensures systematic random sampling through the spinal cord (Fig. 5). Sections have been stored in the deep freezer (target temperature set at -20°C).

### (3) Immunohistology

### Experiment I - GFAP, NeuN and hTDP-43

In Experiment I; glial fibrillary acidic protein (GFAP), neuronal nuclei (NeuN) and hTDP-43 were evaluated on a uniform systematic random set of five sections per mouse brain (one section of L2, L4, L6, L8 and L10; total n = 160 sections) and also on one section of level 3 of the spinal cord tissue array (total n = 32 sections).

### Experiment II - MBP, Iba1 and MAP-2

In Experiment II, myelin basic protein (MBP), ionized calcium-binding adapter molecule 1 (Iba1) and microtubule-associated protein 2 (MAP-2) were evaluated on a uniform systematic random set of five sections per mouse brain (one section of L2, L4, L6, L8 and L10; total n = 160 sections) and also on one section of level 3 of the spinal cord tissue array (total n = 32 sections).

### (4) Imaging

Whole slide scans of the immunofluorescently stained sections were recorded on a Zeiss automatic microscope AxioScan Z1 with high aperture lenses, equipped with a Zeiss Axiocam 506 mono and a Hitachi 3CCD HV-F202SCL camera and Zeiss ZEN 2.3 software.

Immunofluorescently labeled tissue sections of Experiment I and II were analyzed by quantitative image analysis. Image analysis was done with Image Pro 10 (Media Cybernetics). At the beginning the target areas (right hemi brain: cortex and brainstem; spinal cord: left and right ventral horn of cervical, thoracic, and lumbar spinal cord) were identified by drawing regions of interest (ROI) on the images. Additional ROIs exclude wrinkles, air bubbles, or any other artifacts interfering with the measurement. Afterwards, the following signals were quantitatively evaluated within the identified areas:
- GFAP,
- Iba1,
- hTDP-43,
- MBP,
- MAP2,
- NeuN

For quantification we used Edge Plus filtering for background correction. Immunoreactive objects were then detected by adequate thresholding as well as in case of GFAP, Iba1, hTDP-43, and NeuN with additional morphological filtering (size, shape).

Different object features were then quantified among them as the readout:
- Immunoreactive area [%]: The percentage of the ROI that is covered by above-threshold objects (for example: cell somata); this is the most comprehensive parameter indicating whether there are overall differences in immunoreactivity.
- Object density [number of objects per mm²]: The number of above-threshold objects normalized to the size of the target area; this is especially useful to detect changes in the density of immunoreactive objects.
- Object intensity [a.u.]: The average brightness of pixels of above-threshold immunoreactive objects; this indicates if there are differences in the cellular expression level of target proteins.
- Object size [µm²]: The size of above-threshold immunoreactive objects; this is especially useful to detect changes in the size of immunoreactive objects.
- ROI Intensity [a.u.]: The average brightness of pixels within the ROI; this indicates whether there are overall differences in immunofluorescent signal

Once the parameters of the targeted objects have been defined in a test run, the quantitative image analysis runs automatically so that the results are operator-independent and fully reproducible.

Besides the region size of the ROIs investigated, the following data were provided for the respective readouts (for brain and spinal cord areas):
- GFAP: Mean Object Size [µm²], Mean Object Intensity [a.u.], Object Density [n/mm²], Immunoreactive Area [%]
- Iba1: Mean Object Size [µm²], Mean Object Intensity [a.u.], Object Density [n/mm²], Immunoreactive Area [%]
- hTDP-43: Mean Object Size [µm²], Mean Object Intensity [a.u.], Object Density [n/mm²], Immunoreactive Area [%]
- MBP: ROI Mean Intensity [a.u.], Immunoreactive Area [%]
- MAP2: ROI Mean Intensity [a.u.], Immunoreactive Area [%]
- NeuN: Mean Object Size [µm²], Mean Object Intensity [a.u.], Object Density [n/mm²], Immunoreactive Area [%]

### 6-6. Protein Expression Analysis

### (1) Sample preparation

The frozen, unfixed tissue samples (hippocampus of all 15 animals per group and spinal cord of 7 animals per group) were homogenized in 5 volumes per weight RIPA buffer (50 mM Tris-HCl, pH 7.4, 1 mM EDTA, 150 mM NaCl, 1% NP-40 or 0.1% Triton-X, 2% SDS, 1 µM NaF, 0.2 mM sodium deoxycholate, 80 µM glycerophosphate 1 × protease inhibitor (Calbiochem, Germany), 1 × phosphatase inhibitor. Protein concentration was determined by BCA assay. Homogenates were stored at -80°C until further use.

### (2) Western blot analysis

Equal amounts of proteins of each sample were separated by their molecular weight via SDS-PAGE (sodium dodecyl sulfate - polyacrylamide gel electrophoresis; e.g.: Any kD^{™} Mini-PROTEAN^{®} TGX^{™} Precast Protein gels, BioRad). A pre-stained protein marker on each gel visualized correct separation of the proteins and confirmed the correct protein band size on the western blot films later on. Subsequently, proteins were transferred onto nitrocellulose membranes, blocked with 5% non-fat dry milk in 1 × TBS (Tris-buffered saline) and incubated with an antibody specific for using antibodies human-TDP-43 (1: 1000, Abnova, Germany, H00023435-M01) and β-tubulin isotype III (1:5000, Sigma-Aldrich, USA, T8660). HPR (horseradish peroxidase)-coupled, secondary antibodies raised against the species of the primary antibody, ECL (enhanced chemiluminescence) and C-digit blot scanner (Licor) were used for the visualization and semi-quantification of the desired proteins bands.

### 7. Results

### 7-1. Body Weight

The statistical analyses were performed by comparing the groups over the whole experimental period to evaluate the effects of Muse cells on body weight. Therefore, Repeated Measure ANOVA was performed, and significance level of intergroup factor was evaluated. The results were shown in Figs. 6 and 7.

### (1) Comparison between Group D and Group C, and Group C and Group A

At Week 1 to 12, Group C showed significant difference compared to Group D. Group A did not show significant difference compared to Group C.

### (2) Comparison among Group D and Group C, and Group C and Group B

At Week 1 to7 and Week 8 to 12, Group C showed significantly difference compared to Group D. Group B did not show significant difference compared to Group C in each period.

### 7-2. Hanging-Wire Test

The statistical analyses were performed by comparing the groups over the whole experimental period to evaluate the effects of Muse cells on hanging-wire test. Therefore, Repeated Measure ANOVA was performed, and significance level of intergroup factor was evaluated. The results were shown in Figs. 8 and 9.

### (1) Comparison between Group D and Group C, and Group C and Group A

At Week 0 to 12, Group C showed significant difference compared to Group D. Group A showed significant difference compared to Group C.

### (2) Comparison among Group D and Group C, and Group C and Group B

At Week 0 to 6 and Week 8 to 12, Group C showed significant difference compared to Group D. Group B did not show significant difference compared to Group C in each period.

### 7-3. RotaRod

The results were shown in Fig. 10. In Trial 1 to 3 and Mean at Baseline and Week 12, Group C showed significant decrease compared to Group D. Group A to B did not show significant difference compared to Group C.

### 7-4. Clasping

The results were shown in Fig. 11. At Baseline and Week 12, Group C showed significant decrease compared to Group D. Group A to B did not show significant difference compared to Group C.

### 7-5. Evaluation of Immunofluorescence in Spinal Cord Tissue

The immunofluorescent signal was quantified in the spinal cord (cervical, thoracic and lumbar segments) and statistically evaluated.

7-5-1. hTDP-43

The results were shown in Fig. 12.

### (1) Comparison between Group D and Group C

At mean object intensity, object density and immunoreactive area of cervical and thoracic segments, and at all readouts of lumbar segment, Group C showed significant increase compared to Group D. As the result of 2-way ANOVA, at all readouts, factor: Group showed significant difference.

### (2) Comparison between Group C and Group A to B

At mean object intensity and immunoreactive area of thoracic and lumbar segment, Group A showed significant decrease compared to Group C. As the result of 2-way ANOVA, at mean object intensity, object density and immunoreactive area, factor: Group showed significant difference, therefore performed Dunnett's test, Group A to B showed significant difference compared to Group C.

### 7-5-2. GFAP

The results were shown in Fig. 13.

### (1) Comparison between Group D and Group C

At mean object size, object density and immunoreactive area of cervical segment, and mean object intensity, object density and immunoreactive area of thoracic and lumbar segments, Group C showed significant increase compared to Group D. As the result of 2-way ANOVA, at all readouts, factor: Group showed significant difference.

### (2) Comparison between Group C and Group A to B

At mean object size of cervical segment, Group A to B showed significant decrease compared to Group C. As the result of 2-way ANOVA, at mean object size, factor: Group showed significant difference, therefore performed Dunnett's test, Group A to B showed significant difference compared to Group C.

### 7-5-3. MAP2

The results were shown in Fig. 14.

### (1) Comparison between Group D and Group C

At immunoreactive area of thoracic segment, Group C showed significant increase compared to Group D. As the result of 2-way ANOVA, at mean ROI intensity and immunoreactive area, factor: Group showed significant difference.

### (2) Comparison between Group C and Group A to B

At all readouts, Group A to B did not show significant difference compared to Group C. As the result of 2-way ANOVA, at mean ROI intensity and immunoreactive area, factor: Group showed significant difference, therefore performed Dunnett's test, at mean ROI intensity Group A to B showed significant difference compared to Group C, at immunoreactive area Group A showed significant difference compared to Group C, respectively.

### 7-5-4. Iba-1

The results were shown in Fig. 15.

### (1) Comparison between Group D and Group C

At mean object size of cervical segment, object density of thoracic segment, and object density and immunoreactive area of lumbar segment, Group C showed significant increase compared to Group D. As the result of 2-way ANOVA, at all readouts, factor: Group showed significant difference.

### (2) Comparison between Group C and Group A to B

At all readouts, Group A to B did not show significant difference compared to Group C. As the result of 2-way ANOVA, at mean object intensity, factor: Group showed significant difference, therefore performed Dunnett's test, Group A showed significant difference compared to Group C.

### 7-5-5. NeuN

At object density of cervical segment, Group C showed significant increase compared to Group D. As the result of 2-way ANOVA, at object density, factor: Group showed significant difference. Group A and Group B did not show any significant influence in these changes (data not shown).

### 7-5-6. MBP

At all readouts, Group C did not show significant difference compared to Group D. As the result of 2-way ANOVA, at all readouts, factor: Group did not show significant difference (data not shown).

### 7-6. Evaluation of Immunofluorescence in Brain Tissue

The immunofluorescent signal was quantified in the brain (cerebral cortex and brainstem) and statistically evaluated.

### 7-6-1. hTDP-43

The results were shown in Fig. 16.

At all readout of brainstem and cortex, Group C showed a significant increase compared to Group D. Group A to B did not show significant differences compared to Group C.

### 7-6-2. GFAP

The results were shown in Fig. 17.

At object density and immunoreactive area of brainstem, and all readouts of cortex, Group C showed a significant increase compared to Group D. Group A to B did not show significant differences compared to Group C.

### 7-6-3. MAP2

At all readouts of brainstem and cortex, Group C did not show significant differences compared to Group D (data not shown).

### 7-6-4. Iba-1

The results were shown in Fig. 18.

At all readouts of brainstem, and mean object intensity of cortex, Group C showed a significant increase compared to Group D. Group A to B did not show significant differences compared to Group C.

### 7-6-5. NeuN

The results were shown in Fig. 19.

At mean object size of brainstem, Group C showed a significant decrease compared to Group D, and at object density of cortex, Group C showed a significant increase compared to Group D. At all readouts, Group A to B did not show significant differences compared to Group C.

### 7-6-6. MBP

At all readouts of brainstem and cortex, Group C did not show significant differences compared to Group D (data not shown).

### 7-7. Western Blot Analysis

TDP-43 levels determined by Western blotting were quantified in the spinal cord and hippocampus, and statistically evaluated. In the spinal cord and hippocampus, TDP-43/β-Tubulin isotype III, Group C showed significant increases compared to Group D. Group A to B did not show significant differences compared to Group C (Fig. 20).

### Discussion

This study is the first reporting showing that a single intravenous administration of Muse cells at 8 weeks of age improves the results of the hanging-wire test in ALS model hTDP-43 Tg mice. Immunohistological evaluation revealed that hTDP-43 positive reaction, which increased in hTDP-43Tg mice, decreased in the spinal cord. Therefore, it is conceivable that the administration of Muse cells inhibited the aggregation of hTDP-43 in the cytoplasm of nerve cells in the spinal cord and improved motor function.

### Immunohistological analysis

Of the right hemibrains prepared in 6-5 (1), frozen sections are prepared in the same way as in (2) from the brains of 7 cases that are not used for the immunohistological analysis described above. Residual samples of spinal cord tissue arrays (n=8 per group) are also used to prepare cryosections in the same manner. Using the prepared sections, ubiquitin (an indicator of TDP-43 protein accumulation in the cytoplasm), translocator protein (an indicator of neuroinflammation in neurodegenerative diseases), and choline acetyltransferase (a marker of primary and secondary motor neurons) are evaluated by fluorescence immunostaining in the same manner as described above.

By evaluating the expression state of these proteins in the brain and spinal cord with immunostaining, the characteristics of the ameliorative action of Muse cells on the pathological condition of ALS model hTDP-43Tg mice (suppressive action on TDP-43 protein accumulation in the cytoplasm, neuroinflammation inhibitory effects and protective effects on motor nerves, etc.) can be further clarified.

### Protein expression analysis

Of the left hemibrains prepared in 6-4 (2), using the parts other than the hippocampus (15 cases) that are not used in the protein expression analysis by Western blotting described above, the nuclear fraction and the cytoplasmic fraction are separated, and Western blots are performed on each fraction using a human-TDP-43 antibody. Since TDP-43-induced neurodegeneration is induced by the transfer of TDP-43 from the nuclear fraction to the cytoplasmic fraction, by investigating the effect of administration of Muse cells on the distribution of TDP-43 in each fraction, it is possible to further clarify the characteristics of the ameliorating action of Muse cells on the pathological condition of ALS model hTDP-43Tg mice.

The entire disclosures of all references set forth above, and of the corresponding application, are hereby incorporated by reference in their entirety.

### REFERENCES

[1] Aoki, M. et al. Mild ALS in Japan associated with novel SOD mutation. Nat Genet 5, 323-324, doi:10.1038/ng1293-323 (1993).
[2] Gurney, M. E. et al. Motor neuron degeneration in mice that express a human Cu,Zn superoxide dismutase mutation. Science 264, 1772-1775, doi:10.1126/science.8209258 (1994).
[3] Rosen, D. R. et al. Mutations in Cu/Zn superoxide dismutase gene are associated with familial amyotrophic lateral sclerosis. Nature 362, 59-62, doi:10.1038/362059a0 (1993).
[4] Arai, T. et al. TDP-43 is a component of ubiquitin-positive tau-negative inclusions in frontotemporal lobar degeneration and amyotrophic lateral sclerosis. Biochem Biophys Res Commun 351, 602-611, doi:10.1016/j.bbrc.2006.10.093 (2006).
[5] Kabashi, E. et al. TARDBP mutations in individuals with sporadic and familial amyotrophic lateral sclerosis. Nat Genet 40, 572-574, doi:10.1038/ng.132 (2008).
[6] DeJesus-Hernandez, M. et al. Expanded GGGGCC hexanucleotide repeat in noncoding region of C9ORF72 causes chromosome 9p-linked FTD and ALS. Neuron 72, 245-256, doi:10.1016/j.neuron.2011.09.011 (2011).
[7] Renton, A. E. et al. A hexanucleotide repeat expansion in C9ORF72 is the cause of chromosome 9p21-linked ALS-FTD. Neuron 72, 257-268, doi:10.1016/neuron.2011.09.010 (2011).
[8] Abe, K. et al. Exploratory double-blind, parallel-group, placebo-controlled study of edaravone(MCI-186) in amyotrophic lateral sclerosis (Japan ALS severity classification: Grade 3, requiring assistance for eating, excretion or ambulation). Amyotroph Lat Scl Fr 18, 40-48, doi:10.1080/21678421.2017.1361441 (2017).
[9] Writing, G. & Edaravone, A. L. S. S. G. Safety and efficacy of edaravone in well defined patients with amyotrophic lateral sclerosis: a randomised, double-blind, placebo-controlled trial. Lancet Neurol 16, 505-512, doi:10.1016/S1474-4422(17)30115-1 (2017).
[10] Kuroda, Y et al. Unique multipotent cells in adult human mesenchymal cell populations. Proc Natl Acad Sci USA 107, 8639-8643, doi:10.1073/pnas.0911647107 (2010).
[11] Kuroda, Y et al. Isolation, culture and evaluation of multilineage-differentiating stress-enduring (Muse) cells. NatProtoc 8, 1391-1415, doi:10.1038/nprot.2013.076 (2013).
[12] Tanaka, T. et al. Mobilized Muse Cells After Acute Myocardial Infarction Predict Cardiac Function and Remodeling in the Chronic Phase. Circ J 82, 561-571, doi:10.1253/circj.CJ-17-0552 (2018).
[13] Wakao, S. et al. Multilineage-differentiating stress-enduring (Muse) cells are a primary source of induced pluripotent stem cells in human fibroblasts. Proc Natl Acad Sci USA 108, 9875-9880, doi:10.1073/pnas.1100816108 (2011).
[14] Fujita, Y et al. Intravenous injection of Muse cells as a potential therapeutic approach for epidermolysis bullosa. J Invest Dermatol, doi:10.1016/j.jid.2020.05.092 (2020).
[15] Iseki, M. et al. Muse Cells, Nontumorigenic Pluripotent-Like Stem Cells, Have Liver Regeneration Capacity Through Specific Homing and Cell Replacement in a Mouse Model of Liver Fibrosis. Cell Transplant 26, 821-840, doi:10.3727/096368916X693662 (2017).
[16] Uchida, H. et al. Human Muse Cells Reconstruct Neuronal Circuitry in Subacute Lacunar Stroke Model. Stroke 48, 428-435, doi:10.1161/STROKEAHA.116.014950 (2017).
[17] Uchida, N. et al. Beneficial Effects of Systemically Administered Human Muse Cells in Adriamycin Nephropathy. JAm Soc Nephrol 28, 2946-2960, doi:10.1681/ASN.2016070775 (2017).
[18] Yamada, Y et al. S1P-S1PR2 Axis Mediates Homing of Muse Cells Into Damaged Heart for Long-Lasting Tissue Repair and Functional Recovery After Acute Myocardial Infarction. Circ Res 122, 1069-1083, doi:10.1161/CIRCRESAHA.117.311648 (2018).
[19] Yabuki, H., Wakao, S., Kushida, Y, Dezawa, M. & Okada, Y Human Multilineage-differentiating Stress-Enduring Cells Exert Pleiotropic Effects to Ameliorate Acute Lung Ischemia-Reperfusion Injury in a Rat Model. Cell Transplant 27, 979-993, doi:10.1177/0963689718761657 (2018).
[20] Dezawa, M. Clinical trials of Muse cells. Adv. Exp. Med. Biol. 1103, 305-307, doi:10.1007/978-4-431-56847-6_17 (2018).
[21] Noda, T., Nishigaki, K. & Minatoguchi, S. Safety and Efficacy of Human Muse Cell-Based Product for Acute Myocardial Infarction in a First-in-Human Trial. Circ J, doi:10.1253/circj.CJ-20-0307 (2020).
[22] Hayase, M. et al. Committed neural progenitor cells derived from genetically modified bone marrow stromal cells ameliorate deficits in a rat model of stroke. J Cerebr Blood F Met 29, 1409-1420, doi:10.1038/jcbfm.2009.62 (2009).
[23] Chen, Y, Imai, H., Ito, A. & Saito, N. Novel modified method for injection into the cerebrospinal fluid via the cerebellomedullary cistern in mice. Acta Neurobiol Exp (Wars) 73, 304-311 (2013).
[24] Ohta, Y et al. Therapeutic benefits of intrathecal protein therapy in a mouse model of amyotrophic lateral sclerosis. J Neurosci Res 86, 3028-3037, doi:10.1002/jnr.21747 (2008).
[25] Saito, K. et al. Luminescent proteins for high-speed single-cell and whole-body imaging. Nat Commun 3, 1262, doi:10.1038/ncomms2248 (2012).
[26] Abe, T. et al. Intravenously Transplanted Human Multilineage-Differentiating Stress-Enduring Cells Afford Brain Repair in a Mouse Lacunar Stroke Model. Stroke 51, 601-611, doi:10.1161/STROKEAHA.119.026589 (2020).
[27] Vinsant, S. et al. Characterization of early pathogenesis in the SOD1(G93A) mouse model of ALS: part I, background and methods. Brain Behav 3, 335-350, doi:10.1002/brb3.143 (2013).
[28] Abe, K., Morita, S., Kikuchi, T. & Itoyama, Y Protective effect of a novel free radical scavenger, OPC-14117, on wobbler mouse motor neuron disease. J Neurosci Res 48, 63-70 (1997).
[29] Hosaka, Y et al. Alpha1-syntrophin-deficient skeletal muscle exhibits hypertrophy and aberrant formation of neuromuscular junctions during regeneration. J Cell Biol 158, 1097-1107, doi:10.1083/jcb.200204076 (2002).
[30] Nagano, I. et al. Therapeutic benefit of intrathecal injection of insulin-like growth factor-1 in a mouse model of Amyotrophic Lateral Sclerosis. J Neurol Sci 235, 61-68, doi:10.1016/j.jns.2005.04.011 (2005).
[31] Manabe, Y et al. Glial cell line-derived neurotrophic factor protein prevents motor neuron loss of transgenic model mice for amyotrophic lateral sclerosis. Neurol Res 25, 195-200, doi:10.1179/016164103101201193 (2003).
[32] Ohta, Y et al. Neuroprotective and Angiogenic Effects of Bone Marrow Transplantation Combined With Granulocyte Colony-Stimulating Factor in a Mouse Model of Amyotrophic Lateral Sclerosis. Cell Med 2, 69-83, doi:10.3727/215517910X582779 (2011).
[33] Bossolasco, P. et al. Metalloproteinase alterations in the bone marrow of ALS patients. J Mol Med (Berl) 88, 553-564, doi:10.1007/s00109-009-0584-7 (2010).
[34] Kondo, T. et al. Focal transplantation of human iPSC-derived glial-rich neural progenitors improves lifespan of ALS mice. Stem Cell Reports 3, 242-249, doi:10.1016/j.stemcr.2014.05.017 (2014).
[35] Miyazaki, K. et al. Disruption of neurovascular unit prior to motor neuron degeneration in amyotrophic lateral sclerosis. J Neurosci Res 89, 718-728, doi:10.1002/jnr.22594 (2011).
[36] Yamanaka, K. et al. Astrocytes as determinants of disease progression in inherited amyotrophic lateral sclerosis. Nat Neurosci 11, 251-253, doi:10.1038/nn2047 (2008).

## Claims

1. A cell preparation for treating, preventing, alleviating and/or delaying the onset of motor neuron diseases (MNDs) in a subject, comprising pluripotent stem cells positive for SSEA-3 isolated from mesenchymal tissue of a body or cultured mesenchymal cells.

2. The cell preparation according to claim 1, comprising a cell fraction wherein the pluripotent stem cells positive for SSEA-3 have been concentrated by external stress treatment.

3. The cell preparation according to claim 1 or 2, wherein the pluripotent stem cells are CD105-positive.

4. The cell preparation according to any one of claims 1 to 3, wherein the pluripotent stem cells are CD117-negative and CD146-negative.

5. The cell preparation according to any one of claims 1 to 4, wherein the pluripotent stem cells are CD117-negative, CD146-negative, NG2-negative, CD34-negative, vWF-negative, and CD271-negative.

6. The cell preparation according to any one of claims 1 to 5, wherein the pluripotent stem cells are CD34-negative, CD117-negative, CD146-negative, CD271-negative, NG2-negative, vWF-negative, Sox10-negative, Snai1-negative, Slug-negative, Tyrp1-negative, and Dct negative.

7. The cell preparation according to any one of claims 1 to 6, wherein the pluripotent stem cells have all of the following properties:
(i) low or no telomerase activity;
(ii) having the ability to differentiate into any of three germ layers;
(iii) exhibiting non-tumorigenic proliferation; and
(iv) having self-renewal ability.

8. The cell preparation according to any one of claims 1 to 7, wherein the MNDs are amyotrophic lateral sclerosis (ALS), primary lateral sclerosis (PLS), spinal muscular atrophy (SMA), progressive muscular atrophy (PMA), or spinal and bulbar muscular atrophy (SBMA).

9. The cell preparation according to any one of claims 1 to 8, wherein the pluripotent stem cells have the ability to engraft into the spinal cord.
